(19) Europäisches Patentamt
European Patent Office
Office européen des brevets

(11) **EP 4 650 436 B1**

(12) **EUROPEAN PATENT SPECIFICATION**

(45) Date of publication and mention
of the grant of the patent:
**01.07.2026 Bulletin 2026/27**

(51) International Patent Classification (IPC):
**C12N 1/205** (2026.01) **C12N 1/20** (2026.01)
**A01N 63/27** (2020.01) **A01P 21/00** (2006.01)
**C12R 1/38** (2006.01)

(21) Application number: **24176045.3**

(22) Date of filing: **15.05.2024**

(52) Cooperative Patent Classification (CPC):
**C12N 1/205; A01N 63/27; A01P 21/00; C12N 1/20;**
C12R 2001/38

(54) **NOVEL PSEUDOMONAS STRAIN**

NEUER PSEUDOMONAS STAMM

NOUVELLE SOUCHE DE PSEUDOMONAS

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**19.11.2025 Bulletin 2025/47**

(73) Proprietor: **Nordic microbes A/S**
**5000 Odense C (DK)**

(72) Inventors:
• **Vangsøe, Cecilie Toft**
**5230 Odense M (DK)**
• **Hauwaert, Elvira Laila Grud Skat Van**
**5000 Odense C (DK)**
• **Wishoff, Maibrith**
**5210 Odense NV (DK)**
• **Salimi, Zahra**
**5260 Odense S (DK)**

(74) Representative: **Plougmann Vingtoft a/s**
**Strandvejen 70**
**2900 Hellerup (DK)**

(56) References cited:
**WO-A1-2008/130701**

• **DATABASE EMBL [online] 30 April 2024
(2024-04-30), "Pseudomonas alkylphenolica
strain IMGN1 chromosome, complete genome.",
XP93215125, retrieved from EBI accession no.
EM_STD:CP152293 Database accession no.
CP152293**
• **RAFIKOVA G F ET AL: "New Bacterial Strains of
Pseudomonas laurentiana: Promising Agents for
Agrobiotechnology", MOSCOW UNIVERSITY
BIOLOGICAL SCIENCES BULLETIN, vol. 75, no.
4, 2020, pages 206 - 211, XP037360881, ISSN:
0096-3925, DOI: 10.3103/S0096392520040082**
• **RUTZ DOMINIK ET AL: "Comparative Genomic
Analysis of the Biotechnological Potential of the
Novel Species Pseudomonas wadenswilerensis
CCOS 864T and Pseudomonas reidholzensis
CCOS 865T", DIVERSITY, vol. 11, no. 11, 28
October 2019 (2019-10-28), pages 204,
XP093215057, ISSN: 1424-2818, DOI: 10.3390/
d11110204**

Remarks:
The complete document including Reference
Table(s) and the Sequence Listing(s) can be
downloaded from the EPO website

## Description

### Technical field of the invention

The present invention relates to isolated bacteria or biologically pure bacterial culture of the genus *Pseudomonas*. The invention also relates to compositions such as fertilizers, probiotics, medicines, and seed coatings comprising said bacteria and uses thereof, in particular uses as plant growth promoting agents in colder regions.

### Background of the invention

The United Nations estimate that we will reach 8 billion people this year, and nearly 10 billion people by 2050. This population growth, and a parallel rise in wealth, will increase food demand by 50% in 2050. In the same period, the agricultural area per capita will decrease by about 25%. The lack of food is already urgent, the World Food Programme estimates that, in 2022, 828 million people do not have enough food and 50 million are facing emergency levels of hunger around the world. The amount of people that are already malnourished, the projected growth in population and food consumption and the decrease in farmland per capita highlights the need for a more efficient and productive agricultural industry.

Agrochemicals, such as fertilizers and pesticides, are the primary means by which agricultural productivity is increased currently. The widespread use of these chemicals has negative environmental consequences. They can affect the health of humans, animals, plants, soils and microbiomes, they can degrade ecosystems, promote anti-microbial resistance and their use contributes to climate change. Further increasing the use of these chemicals to support the demands for higher food productivity would therefore be highly problematic.

The germination, emergence, growth, development, yield quantity and quality of a plant may be beneficially affected by microorganisms that enhance the availability of macronutrients and micronutrients, strength plant defences and protect them against biotic and abiotic stress factors. Due to global warming and past agricultural practices, there is an increase in more extreme weather, drought, floods, heat waves, salt stress, pollution of soil and water sources and a loss of soil fertility and health. There is also an increase and spread of new plant pathogens and anti-microbial resistance. The addition of beneficial bacteria to plants may be a method for increasing food productivity under normal conditions, but it may also be a method for decreasing losses due to extreme weather and other biotic and abiotic stress conditions.

There are many mechanisms by which beneficial microorganisms may promote plant growth or survival. Beneficial bacteria help the plant access heavily bound nutrients in the soil that would otherwise not be accessible. They may increase root size or branching and improve the availability of water and nutrients for the plant. The bacteria may also bind water and nutrients and provide it to the plant during periods of drought or increased nutrient needs. Bacteria may also colonize the surface of plants and seeds such as their leaves and roots and protect it against unfriendly microorganisms through specific mechanisms or by simply taking the space and nutrients that pathogenic bacteria and fungi may have used. Not all bacteria are beneficial to plants, however, some may be directly detrimental to plant growth whereas others may not damage the plant directly but still affect them indirectly by taking space and nutrients that would otherwise be available to beneficial microorganisms. Making sure that beneficial bacteria are present on, in and near plants has potential as a solution for increasing agricultural productivity of a given area of farmland without adding further agrochemicals. Bacteria that are beneficial to plants may also have applications in other fields than agriculture such as in horticulture, vertical farming, gardens, landscaping, reforestation and in the restoration of degraded areas. In addition, bacteria that are capable of solubilizing nutrient-containing minerals may have applications outside agriculture such as in promoting the health of humans and animals as well as in mining, ore extraction, and waste reuse/recycling.

WO 2020/214843 A1 discloses a method of incorporating *Pseudomonas* bacteria into a plant seed for increasing plant growth, improving nutrient supply, enhancing plant defence against plant pathogens such as fungi and viruses, and improving tolerance to abiotic stress. The growth temperatures of the plant seeds are disclosed as 19-25°C. However, the *Pseudomonas* bacteria have not been reported to be active under low-temperature conditions, such as below 20°C and even below 7°C, representing the climate in Scandinavia during mild winter or spring seasons.

Rafikova et al. ("New Bacterial Strains of Pseudomonas laurentiana: Promising Agents for Agrobiotechnology", MOSCOW UNIVERSITY BIOLOGICAL SCIENCES BULLETIN, 2020, vol. 75, no. 4, pages 206-211) discloses *Pseudomonas laurentiana* strains, which possess a set of properties of plant growth-promoting microorganisms, e.g. antifungal activity against phytopathogenic fungi and the ability of decomposing phosphates.

WO 2008/130701 A1 discloses a *Pseudomonas antarctica* and a *Pseudomonas trivialis* strain, which are able to solubilize phosphorus, as well as seeds coated with the strains and the use of the strains as biofertilizer.

Hence, expanding the portfolio of bacteria and providing a bacteria capable of improving plant growth, such as at low temperatures, would be advantageous, and in particular, more efficient and/or reliable compositions comprising such bacteria would be advantageous.

## Summary of the invention

The inventing team has isolated, identified, characterized and propagated a newly identified strain of bacteria, namely the *Pseudomonas* strain DSM 34653 deposited with deposit number DSM 34653. The strain may be used to promote a wide variety of plant health parameters. Such parameters may for example be the speed by which seeds germinate, the percentage of seeds that germinate, the speed by which root or shoot elongates and grows, the speed by which sown seeds emerge, the percentage of sown seeds that emerge, the speed by which a plant grows, the speed by which a plant develops on the BBCH scale, the size and shape of particular parts of the plant e.g. roots, stem, leaves, flowers and seeds, the colors of a plant or parts of the plant, the resistance of the plant towards biotic and abiotic stresses, the appearance, taste and smell of a plant as well as the yield (mass/area) and yield properties (e.g. protein, starch, oil or toxin content). Promoting these parameters may have application in agriculture, horticulture, landscaping, gardening, and indoor plants, users may be business or private individuals and the plants may be food crops, fiber crops, fuel crops or plants that serve aesthetic or functional purposes such as flowers, grass and trees. Promoting these parameters may have application in the restoration of degraded land whereon plant cover is needed, such as degraded mine sites or degraded agricultural or pastoral land. It may also have applications in reforestation or revegetation and in carbon capture/storage based on growing biomass.

The strain may also be used to inhibit the growth of pathogens, such as bacteria and fungi. This may have application in promoting the health of plants and potentially also humans.

The strain may also be used to dissolve otherwise poorly soluble substances such as minerals and salts. These minerals and salts may be present naturally in soil or other plant growth media, examples are calcium phosphate, iron phosphate, aluminium phosphate and potassium alumino silicate. They may also form when macronutrients or micronutrients, possibly in the form of fertilizer, are added to the soil or other plant growth media. The minerals and salts may also be components of nutrient bearing materials destined for agricultural use such as wastewater, sludge, biochar, manure, green manures, compost, biogas and pyrolysis residues, biowaste, household waste and other biomasses. The purpose of adding the strain would in these cases be to dissolve the minerals or salts and thereby release nutrients such as phosphate, potassium, calcium, magnesium, sulfate, manganese and iron that can enhance plant growth.

The strain may also be used to dissolve otherwise poorly soluble substances such as minerals and salts for applications outside plants. It may be used to bioleach ores or waste materials. It may therefore be used in mining, recycling, or urban mining.

Example 1 shows how bacterial strains with biocontrol properties were isolated from the soil of a Danish grassland and screened to identify the best-performing bacterial strain. The microbeTRAP enabled capture of multiple potential bacterial biocontrol strains from Danish grassland soil. Of the isolated strains, TF-D (DSM 34653) was chosen as the most promising candidate for a biocontrol agent based on the initial screening for biocontrol properties on three common phytopathogenic fungi.

Example 2 shows that DSM 34653 is a novel bacterial species belonging to the genus *Pseudomonas.* Interestingly, DSM 34653 encodes several properties that are beneficial to agricultural farming, including phosphate solubilizing capabilities. As DSM 34653 is classified as a novel bacterial species, virulence and antimicrobial resistance properties were assessed. These analyses revealed the presence of few virulence and antimicrobial resistance genes.

Several DNA sequences containing open reading frames and that have high specificity for DSM 34653 were identified; three long genomic regions (14,515-16,414 nucleotides, SEQ ID NOs: 2-4) wherein short DNA sequences (303-2,779 nucleotides) were extracted (SEQ ID NOs: 5-13) as well as four short DNA sequences (714-1,077 nucleotides) identified outside of the three long genomic regions (SEQ ID NOs: 14-17). Combined or alone, these DNA sequences may be used to identify DSM 34653 at the DNA sequence level.

Example 3 shows the ability of DSM 34653 to control growth of common fungal plant pathogens. DSM 34653 was shown to have antifungal activity against four common phytopathogenic fungi *Gaeumannomyces graminis var. tritici* (isolate CBS 450.77), *Pyrenophora teres f. teres* (isolate CP2189), *Zymoseptoria tritici* (isolate IPO323), *Fusarium oxysporum* (isolate CBS 619.87) when evaluated in a dual culture assay. Based on these results DSM 34653 could hold potential for mitigating crop losses caused by fungal diseases, thereby enhancing agricultural productivity and sustainability.

Example 4 shows the efficacy of DSM 34653 as a biostimulant in increasing the availability of common plant nutrients under low, medium, and high temperature conditions. Only DSM 34653 was able to grow and display bacterial activity at 5°C, demonstrating that DSM 34653 has a high potential for supplying crops in the field with otherwise unavailable nutrients through the entire plant life cycle from as early as germination in cold soil until maturation and harvest.

Example 5 shows the stimulatory properties of DSM 34653, when applied as a seed coat, on early plant growth *in vitro* in spring barley. Treating spring barley seeds with a bacterial solution containing DSM 34653 before sowing demonstrates that DSM 34653 can enhance the emergence of seedlings, also when seeds have been treated with a commercially available fungicide, suggesting that an increased yield also may be obtained.

The aforementioned deposit was made by Bioomix A/S on 24 May 2023. The deposit was given the following reference number: DSM 34653.

Thus, an object of the present invention relates to the provision of novel bacterial strains/biostimulants for improving plant growth, such as at low temperatures.

In particular, it is an object of the present invention to provide novel bacterial strains/biostimulants for improving plant growth that can be effective at colder soil or air temperatures such as below 10°C.

Thus, one aspect of the present invention relates to a bacteria or biologically pure bacterial culture

- being *Pseudomonas,* DSM 34653 deposited with the DSMZ [LEIBNIZ-INSTITUT DSMZ-DEUTSCHE SAMMLUNG VON MIKROORGANISMEN UND ZELLKULTUREN GmbH, Inhoffenstr. 7 B, D-38124, Braunschweig, Germany] on 24 May 2023.

Yet another aspect of the invention relates to a fertilizer and/or inoculant and/or biostimulant and/or biofungicide and/or antimicrobial composition comprising the bacteria or biologically pure bacterial culture according to the invention.

A further aspect of the present invention relates to a coating composition, preferably a seed coating composition, comprising the bacteria or biologically pure bacterial culture according to the invention and/or the composition according to the invention.

Another aspect of the present invention relates to a plant seed coated with the composition according to the invention or coated with a coating composition according to the invention.

Yet another aspect of the present invention relates to use of a bacteria or biologically pure bacterial culture according to the invention, the composition according to the invention or the coating composition according to the invention, as a plant growth promoting agent, such as a fertilizer or inoculum or biostimulant.

Another aspect of the present invention relates to a method for stimulating plant growth comprising applying the bacteria or biologically pure bacterial culture according to the invention, the composition according to the invention, or the coating composition according to the invention to a plant, plant seed, a sowing furrow, soil and/or plant growth medium.

A further aspect of the present invention relates to a kit of parts for stimulating plant growth comprising

- a first container comprising the bacteria or biologically pure bacterial culture according to the invention, the composition according to the invention and/or the coating composition according to the invention; and
- instructions for applying the bacteria or biologically pure bacterial culture according to the invention, the composition according to the invention and/or the coating composition according to the invention to plants, plant seeds, or a plant growth medium.

A yet an aspect of the present invention relates to a bacteria or biologically pure bacterial culture according to the invention, a composition according to the invention or a coating composition according to the invention for use as a medicament.

Another aspect relates to a bacteria or biologically pure bacterial culture according to the invention or a composition according to the invention use in the treatment, alleviation and/or prevention of fungal infections.

A yet an aspect of the invention relates to use of the (isolated) bacteria or biologically pure bacterial culture according to the invention or the composition according to the invention for solubilizing minerals in ores.

### Brief description of the figures

Figure 1

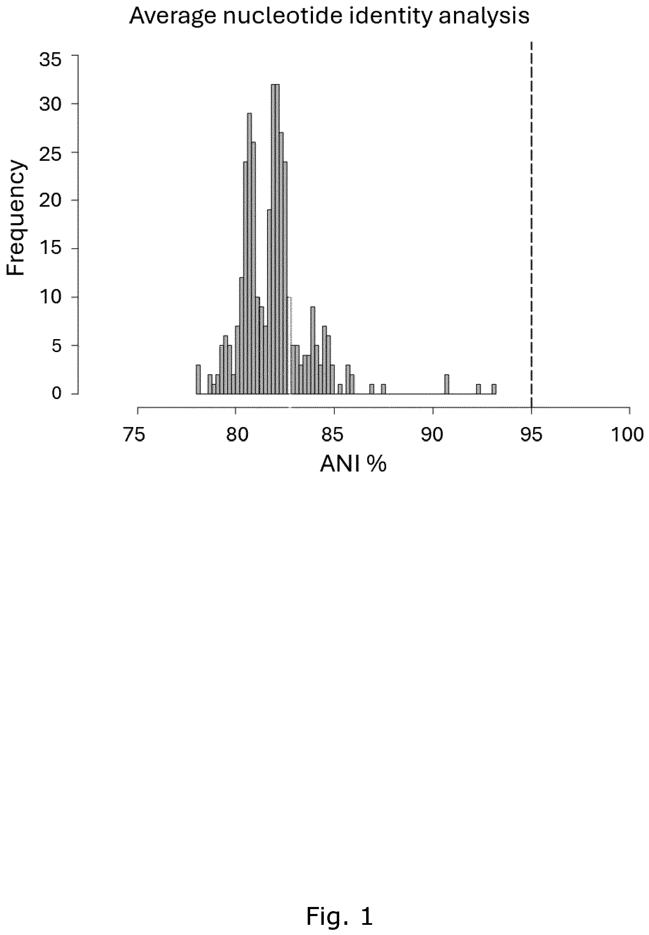

Figure 1 shows a histogram showing genome-to-genome comparisons from an average nucleotide identity (ANI) analysis of the genome assembly representing DSM 34653 and all *Pseudomonas* RefSeq reference genomes available at NCBI (n=368, excluding atypical genomes, as of April 3, 2024). The dotted vertical line represents the boundary for which new species are defined (at 95% ANI).

Figure 2

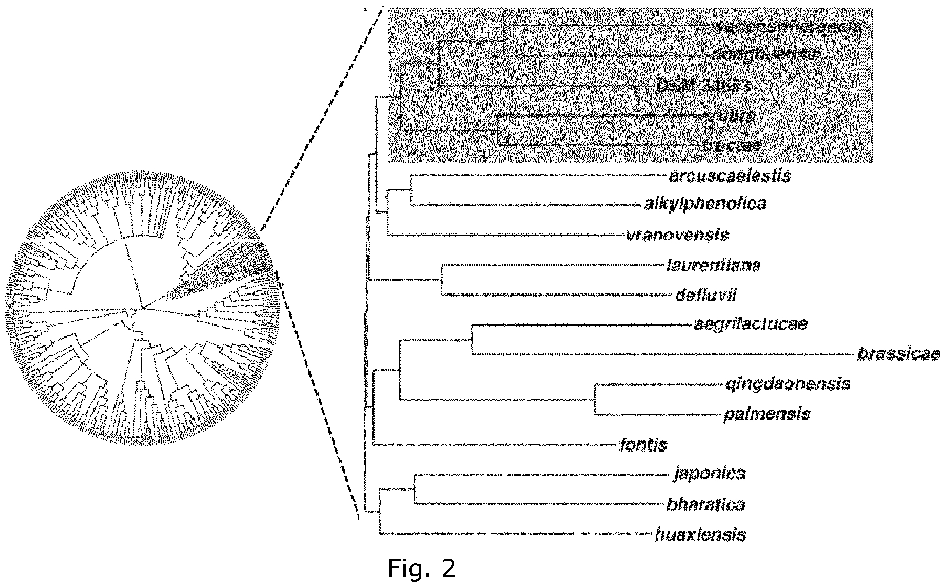

Figure 2 shows phylogenetic trees representing evolutionary relatedness between DSM 34653 and *Pseudomonas* references at species level (all *Pseudomonas* RefSeq reference genomes available at the National Center for Biotechnology Information (NCBI) (n=368, excluding atypical genomes, as of April 3, 2024)) (circular tree to the left). The tree to the right represents a zoomed view of the clade containing DSM 34653 (further highlighted within the grey area).

Figure 3

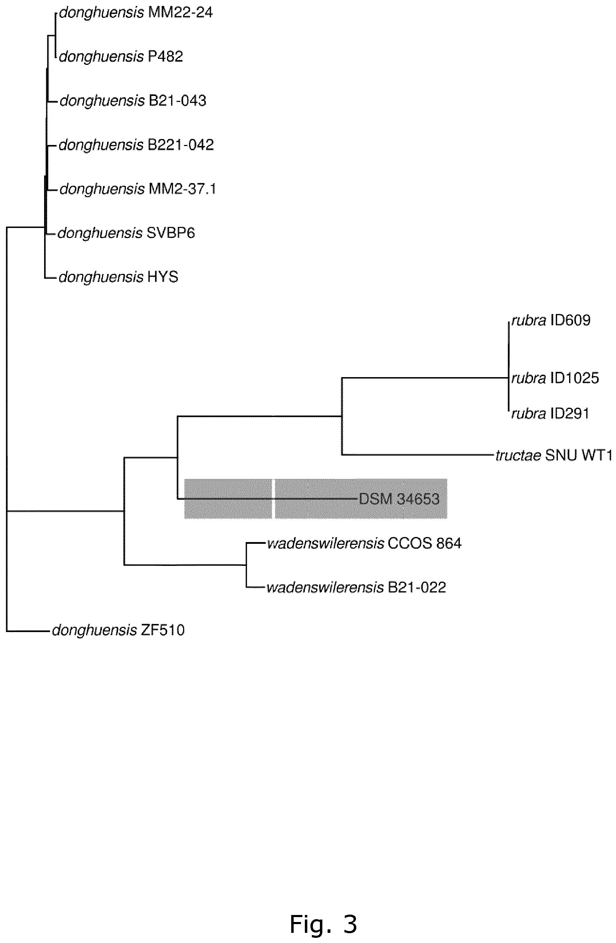

Figure 3 shows a phylogenetic tree representing evolutionary relatedness between DSM 34653 and all available RefSeq strain genomes at the National Center for Biotechnology Information (NCBI) belonging to the following *Pseudomonas* species: *wadenswilerensis, donghuensis, tructae* and *rubra* (n=14, excluding atypical genomes, as of April 5, 2024). DSM 34653 is highlighted within the grey area.

Figure 4

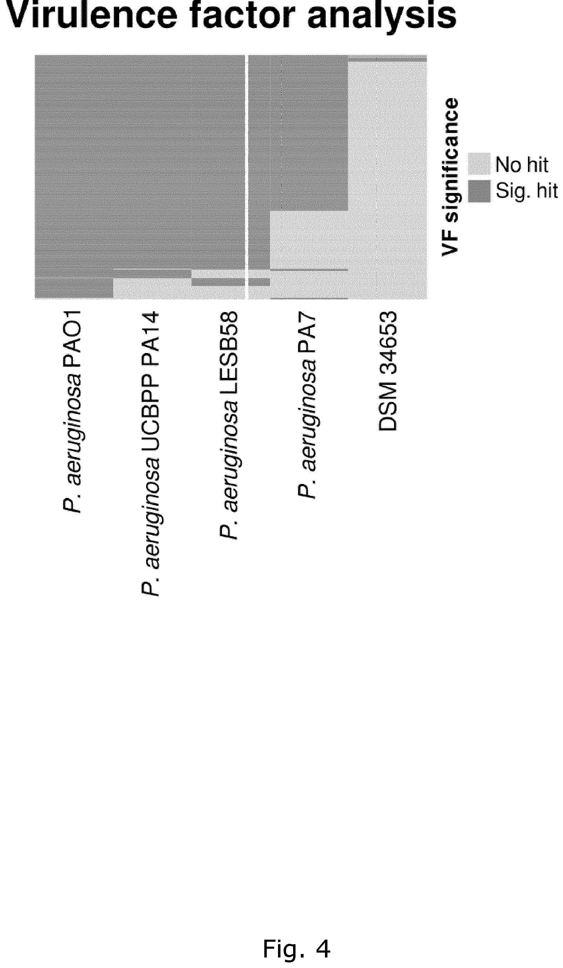

Figure 4 shows a heatmap representing the presence of virulence factors (VFs) in the genome assembly of DSM 34653 and four pathogenic *Pseudomonas aeruginosa* strains (PAO1, UCBPP PA14, LESB58 or PA7). Dark grey bars indicate significant hits (presence of a VF) and light grey bars indicate no hit (no presence of VF in strain). 329 significant and unique VFs were found across all genomes analyzed.

Figure 5

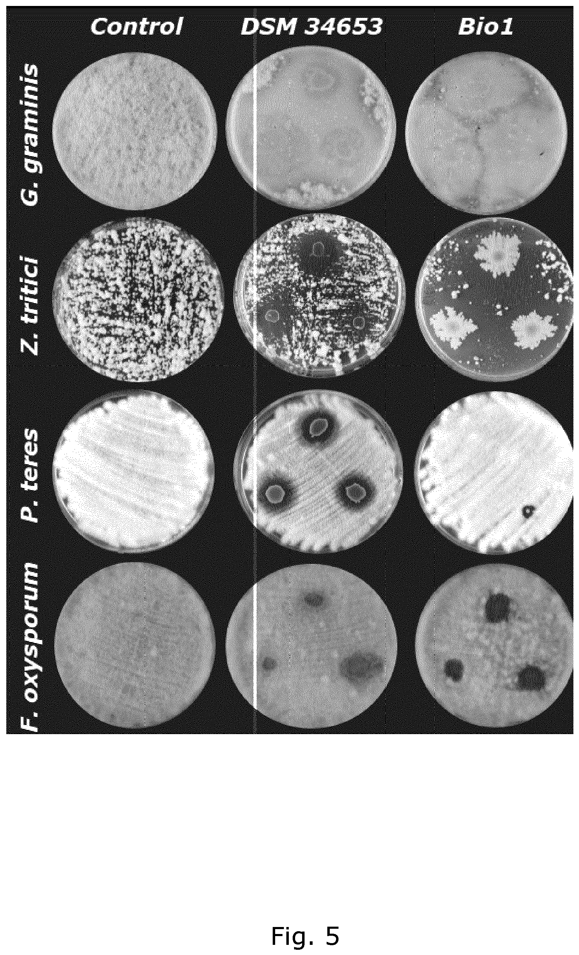

Figure 5 shows the ability of DSM 34653 to inhibit the growth of four common phytopathogenic fungi. Dual cultures in a spot on lawn design with DSM 34653 and a commercial biofungicide (Bio1, *Bacillus amyloliquefaciens* QST 713) on plates covered with *Gaeumannomyces graminis (G. graminis), Zymoseptoria tritici (Z. tritici), Pyrenophora teres f. teres (P. teres),* or *Fusarium oxysporum (F. oxysporum).* Control plates are shown to the left. *G. graminis* and *F. oxysporum* were cultivated on oatmeal agar (OA), *P. teres* on grass agar (GA), and *Z. tritici* on potato dextrose agar (PDA).

Figure 6

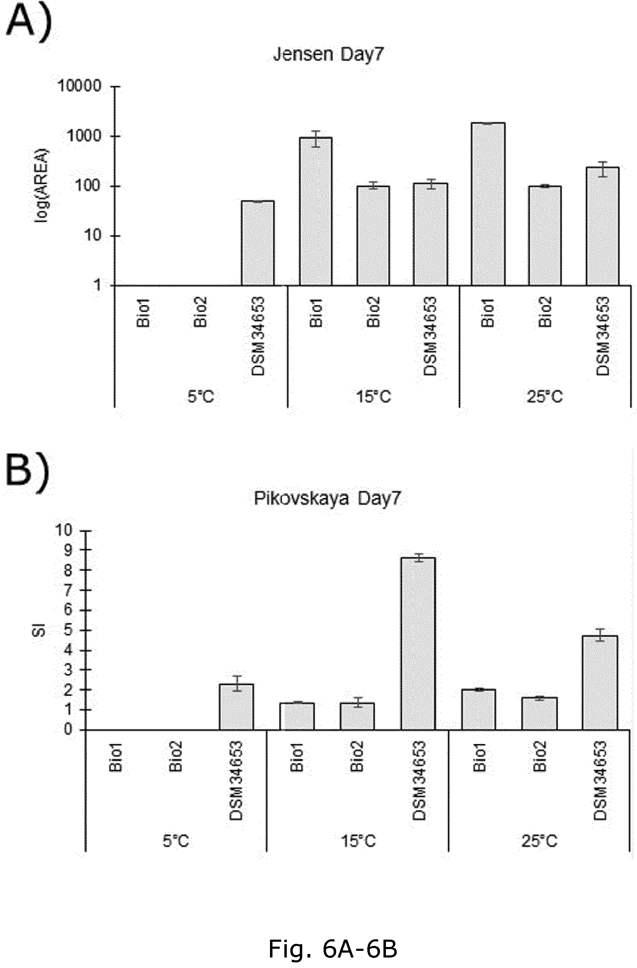

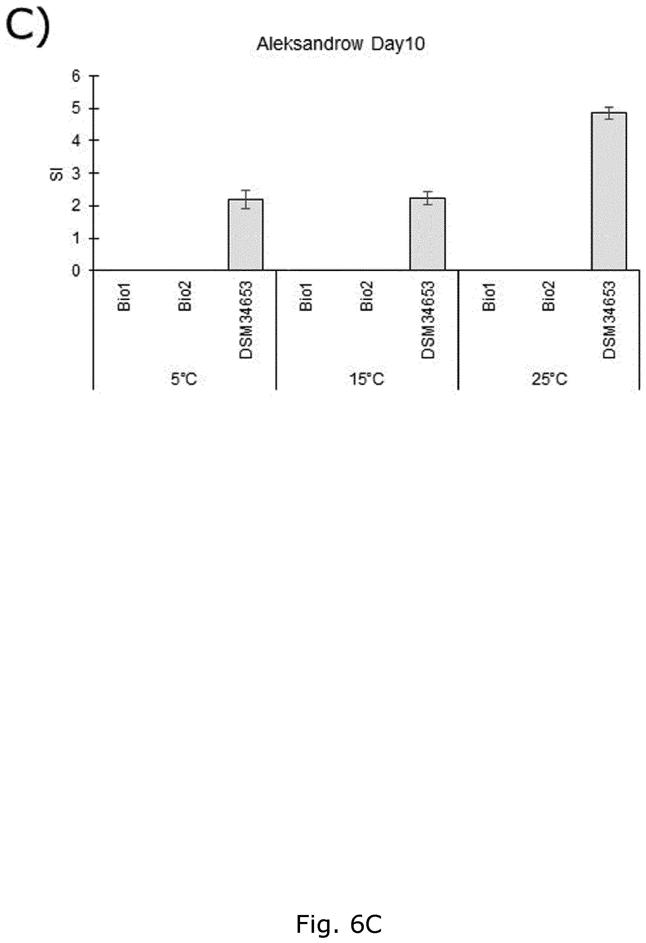

Figure 6 shows *in vitro* mineral-dissolving properties of DSM 34653 and of two strains isolated from commercially available microbial plant products at low, medium and high temperatures. **A)** Colony size of DSM 34653 and of two bacterial strains (Bio1, *Bacillus amyloliquefaciens* QST 713 and Bio2, *Bacillus atrophaeus* Abi05) isolated from one biostimulant and one biofungicide commercially available product that were grown on Jensen medium (nitrogen-free agar) for 7 days at low (5°C), medium (15°C) or high (25°C) temperatures. Colony sizes were quantified on a log scale. **B),** and **C)** Solubilization index (SI, $Area_{Halo}/Area_{Colony}$) for DSM 34653, Bio1 and Bio2 grown on Pikovskaya medium (insoluble phosphate agar) for 7 days (**B**), Aleksandrow medium (insoluble potassium agar) for 10 days (**C**) at low (5°C), medium (15°C) or high (25°C) temperatures. Experiments were carried out in three replicates. Bars in each plot represent the mean across replicates and error bars represent standard deviations across replicates in each experimental group.

Figure 7

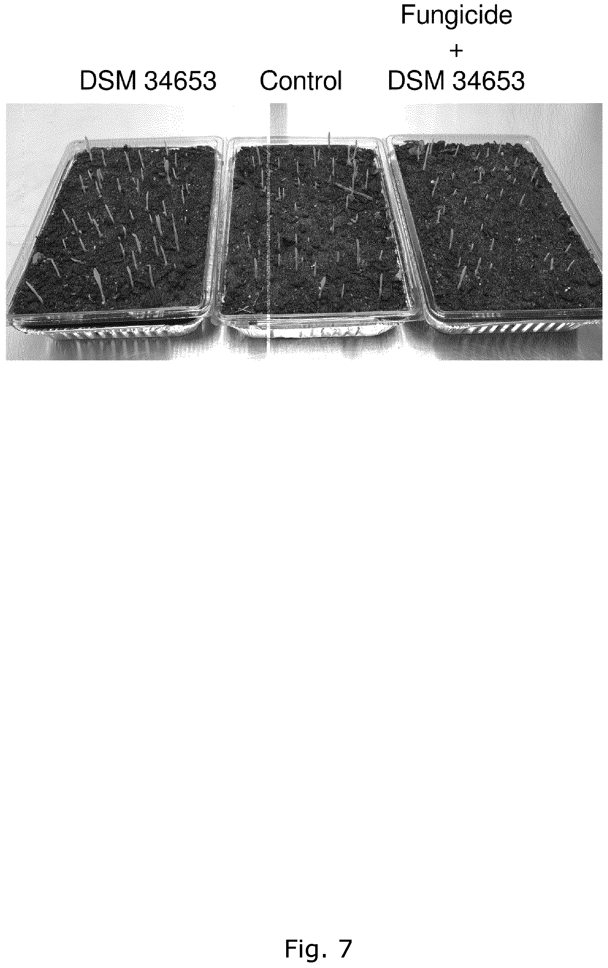

Figure 7 shows the emergence of seedlings from spring barley seeds five days following sowing. 70 seeds were sowed in each experimental group: DSM 34653; seeds coated with a bacterial solution containing DSM 34653, Control; untreated seeds; Fungicide + DSM 34653; seeds coated with a commercially available chemical fungicide (containing tebuconazol (20 g/L) and prothioconazol (150 g/L) as active components) and DSM 34653.

The present invention will now be described in more detail in the following.

**Detailed description of the invention**

Definitions

Prior to discussing the present invention in further details, the following terms and conventions will first be defined:

*Effective amount*

In the present context, the term "effective amount" refers to a quantity which is sufficient to result in a statistically significant increase in a desirable plant property such as germination, emergence, growth and/or of protein yield and/or of grain/crop yield of a plant as compared to the germination, emergence growth, protein yield and grain yield of the control-treated plant.

*Inoculant*

The term "inoculant" as described in this invention is defined in several Federal, or State regulations as:

(1) "soil or plant inoculants shall include any carrier or culture of a specific microorganism or mixture of micro-organisms represented to improve the soil or the growth, quality, or yield of plants, and shall also include any seed or fertilizer represented to be inoculated with such a culture" (New York State 10-A Consolidated Law);

(2) "substances other than fertilizers, manufactured, sold or represented for use in the improvement of the physical condition of the soil or to aid plant growth or crop yields" (Canada Fertilizers Act);

(3) "a formulation containing pure or predetermined mixtures of living bacteria, fungi or virus particles for the treatment of seed, seedlings or other plant propagation material for the purpose of enhancing the growth capabilities or disease resistance or otherwise altering the properties of the eventual plants or crop" (Ad hoc European Working Group, 1997); or

(4) "meaning any chemical or biological substance of mixture of substances or device distributed in this state to be applied to soil, plants or seeds for soil corrective purposes; or which is intended to improve germination, growth, quality, yield, product quality, reproduction, flavor, or other desirable characteristics of plants or which is intended to produce any chemical, biochemical, biological or physical change in soil" (Section 14513 of the California Food and Agriculture Code).

*Biostimulant*

In the present context a "biostimulant" or plant biostimulant is any substance or microorganism applied to plants with the aim to enhance nutrition efficiency, abiotic stress tolerance and/or crop quality traits, regardless of its nutrients content. By extension, plant biostimulants also designate commercial products containing mixtures of such substances and/or microorganisms.

In here the terms "fertilizer", "inoculant" and "biostimulant" may be used interchangeably.

*Fungicide*

In the present context a "fungicide" is a substance used to kill or prevent the growth of fungi and their spores. They can be used to control fungi that damage plants, including but not limited to rusts, mildews, wilts, canker, smuts, damping-off, rot, anthracnose, scab, mould, and blights.

Fungicides may be classified as chemical fungicides or biofungicides.

*Biofungicide*

In the present context a "biofungicide" are formulations of one or more living organisms that are used to control the activity of plant pathogenic fungi and bacteria. The mechanisms used to control plant pathogens include competition, parasitism, antibiosis, induction of plant defense responses, and promotion of plant growth.

*Isolated bacteria and biologically pure bacterial culture*

In the present context, the terms "isolated bacteria" and "biologically pure bacterial culture" refer to isolated bacteria or a culture of bacteria containing no other bacterial species in quantities sufficient to interfere with the replication or function of the culture or be detected by normal bacteriological techniques. Stated another way, it is a culture wherein virtually all of the bacterial cells present are of the selected strain.

Phrased in a different way, the "biologically pure bacterial culture" is at least 90% pure, such as at least 95% pure, such as at least 98% pure, such as at least 99% pure, such as 99.5% pure. Percentage is to be determined by a number of bacteria in the culture.

Preferably the bacteria according to the invention is an isolated bacteria, such as forming part of an isolated composition.

*Plant growth promoting agent*

In the present context, the term "plant growth promoting agent" refers to the ability to enhance or increase at least one desirable plant trait or property such as the plant's height, weight, leaf size, root size, or stem size, to increase protein yield from the plant or to increase grain/crop yield of the plant.

*Sequence identity*

In the present context, the term "sequence identity" indicates a quantitative measure of the degree of homology between two amino acid sequences of substantially equal length or between two nucleic acid sequences of substantially equal length. The two sequences to be compared must be aligned to best possible fit with the insertion of gaps or alternatively, truncation at the ends of the protein sequences. The sequence identity can be calculated as $\dfrac{\left(N_{ref}-N_{dif}\right)100}{N_{ref}}$, wherein $N_{dif}$ is the total number of non-identical residues in the two sequences when aligned and wherein $N_{ref}$ is the number of residues in one of the sequences. Hence, the DNA sequence AGTCAGTC will have a sequence identity of 75% with the sequence AATCAATC ($N_{dif}$=2 and $N_{ref}$=8). A gap is counted as non-identity of the specific residue(s), i.e. the DNA sequence AGTGTC will have a sequence identity of 75% with the DNA sequence AGTCAGTC ($N_{dif}$=2 and $N_{ref}$=8). Sequence identity can alternatively be calculated by the BLAST program e.g. the BLASTP program for protein alignment (W.R Pearson and D.J. Lipman (1988)).

For calculations of sequence identity when comparing polypeptide fragments with longer amino acid sequences, the polypeptide fragment is aligned with a segment of the longer amino acid sequence. The polypeptide fragment and the segment of the longer amino acid sequence may be of substantially equal length. Thus, the polypeptide fragment and the segment of the longer amino acid sequence may be of equal length. After alignment of the polypeptide fragment with the segment of the longer amino acid sequence, the sequence identity is computed as described above.

A preferred minimum percentage of sequence identity is at least 80%, such as at least 85%, at least 90%, at least 91%, at least 92%, at least 93%, at least 94%, at least 95%, at least 96%, at least 97%, at least 98%, at least 99%, and at least 99.5%.

*Solubilization*

In the present context, the term "solubilization" in relation to mineral solubilization is to be understood as the dissolution of insoluble minerals such as calcium phosphate ($Ca_3(PO_4)_2$), iron phosphate ($FePO_4 \cdot 4\,H_2O$), and potassium alumino-silicate and thereby increase the availability of common plant nutrients. Soil phosphate and potassium exist to a large extent in insoluble complexes bound to insoluble inorganic minerals, which are unavailable for plants. Thus, solubilization/dissolution of these minerals may improve mineral availability for plant uptake.

The level of solubilization can be determined by:

- providing solid media such as agar plates comprising insoluble mineral complexes,
- cultivating bacteria of interest,
- and calculating a solubilization index (SI) using the formula:

$$SI = Area_{Halo}/Area_{Colony}$$

The "Area" is determined by measuring the diameter of the bacterial colonies and the corresponding clear/halo zones.

**Bacteria or biologically pure bacterial culture**

As outlined above, the inventing team has identified, isolated and propagated a newly identified strain of bacteria, namely the *Pseudomomas* strain DSM 34653 deposited with deposit number DSM 34653 The strain may be used to promote a wide variety of plant health parameters.

The bacterial strain of the present invention was identified by isolating bacterial strains using microbeTRAP to capture multiple potential bacterial biocontrol strains from Danish grassland soil and screened to identify the best-performing bacterial strain. A comparative screening test was conducted to identify the most effective candidate among the isolated strains (n=7). Of the isolated strains, DSM 34653 was chosen as the most promising candidate for a biocontrol agent based on the initial screening for biocontrol properties on three common phytopathogenic fungi (Example 1).

As shown in Example 2, comparative genome analysis of the bacterial strain DSM 34653 reveals that DSM 34653 is a novel *Pseudomonas* strain.

Thus, in an embodiment, the bacteria or biologically pure bacterial culture according to the invention, comprising,

a) a genomic sequence according to any of SEQ ID NO: 2-4;
b) a genomic sequence having at least 90% sequence identity to any of SEQ ID NO: 2-4, such as at least 95%, such as at least 97%, such as at least 98%, such as at least 99%, such as at least 99.5%, such as at least 99.9 % sequence identity to any of SEQ ID NO: 2-4, and/or
c) a fragment of the sequence of a) or b), having a length of at least 5000 nucleotides, such as at least 8000 nucleotides, such as at least 10000 nucleotides, such as at least 14000 nucleotides.

The DSM 34653 strain according to the invention has been annotated as being a *Pseudomonas* genus. Thus, in an embodiment, the bacteria or biologically pure bacterial culture is of the genus *Pseudomonas*, such as *Pseudomonas wadenswilerensis, Pseudomonas donghuensis, Pseudomonas tructae,* or *Pseudomonas rubra.*

In an embodiment, the bacteria or biologically pure bacterial culture comprises a 16S rRNA gene encoded by SEQ ID NO: 1.

As shown in Example 2, six 16S ribosomal RNA (rRNA) genes were identified in the assembled genome of DSM 34653. These six 16S rRNA gene sequences are 100% identical to each other, the DNA sequence corresponding to one of these is encoded by SEQ ID NO: 1.

In an embodiment, the isolated bacteria or biologically pure bacterial culture being *Pseudomonas,* DSM 34653 deposited with the DSMZ [(LEIBNIZ-INSTITUT DSMZ-DEUTSCHE SAMMLUNG VON MIKROORGANISMEN UND ZELLKULTUREN GmbH, Inhoffenstr. 7 B, D-38124, Braunschweig, Germany) on 24 May 2023]. In the present patent application, this strain is also named TF-D or TF2.

In yet an aspect the invention relates to an (isolated) bacteria or biologically pure bacterial culture being *Pseudomonas,* DSM 34653 deposited with the DSMZ [LEIBNIZ-INSTITUT DSMZ-DEUTSCHE SAMMLUNG VON MIKROORGANISMEN UND ZELLKULTUREN GmbH, Inhoffenstr. 7 B, D-38124, Braunschweig, Germany] on 24 May 2023.

For colder regions it would be an advantage if the bacteria is active under colder conditions. Thus, in an embodiment, the bacteria or biologically pure bacterial culture according to the invention is

- capable of solubilizing calcium phosphate at 5°C, and/or iron phosphate at 5°C and/or potassium aluminum silicate at 5°C; and/or
- capable of inhibiting one or more phytopathogenic fungi, such as phytopathogenic fungi specific for (colder) regions.

As seen in Example 4, it is evident that the bacteria is capable of solubilizing calcium phosphate ($Ca_3(PO_4)_2$) and potassium alumino-silicate at low (5°C), medium (15°C), and high (25°C) temperatures. Especially, the bacteria of the present invention is able to grow and display bacterial activity at temperatures down to 5°C.

As seen in Example 3, the bacteria has antifungal activity against phytopathogenic fungi *Gaeumannomyces graminis var. tritici, Pyrenophora teres, Zymoseptoria tritici,* and *Fusarium oxysporum.*

Thus, in an embodiment, the one or more phytopathogenic fungi are selected from the group consisting of *Gaeumannomyces graminis var. tritici, Pyrenophora teres f. teres, Zymoseptoria tritici,* and *Fusarium oxysporum.*

In an embodiment, the solubilization of calcium phosphate, and/or iron phosphate, and/or potassium aluminum silicate at 5°C is at a level of at least 40% to, equal to and/or above a level of solubilization at 15°C and/or 25°C.

As seen in Figure 6A-6C, the solubilization of minerals by the bacteria of the present invention is indeed works at a temperature of 5°C and at higher temperatures at 15°C and 25°C.

This demonstrates that the bacteria of the present invention has a high potential for supplying crops in the field with otherwise unavailable nutrients through the entire plant life cycle from as early as germination in cold soil until maturation and harvest.

In an embodiment, the level of solubilization is determined by a solubilization index (SI) calculated by the formula:

$$SI = Area_{Halo}/Area_{Colony}.$$

As seen in Example 4, the solubilization index (SI) is calculated using the above formula.

In an embodiment, the SI is at least 2, such as at least 4, such as between 2 and 10, preferably the SI is between 2 and 9.

As seen in Example 4 the solubilization index (SI) of DSM 34653 on day 7 on Pikovskaya agar was in the range 2 and 9, wherein the SI was about 2.3 at 5°C, about 8.6 at 15°C, and about 4.7 at 25°C **(Figure 6B)**. The SI of DSM 34653 on day 10 on Aleksandrow agar was in the range 2 and 5, wherein the SI was about 2.2 at 5°C, about 2.2 at 15°C, and about 4.8 at 25°C **(Figure 6C)**.

In a further embodiment, the SI depends on temperature after cultivation start and/or incubation time.

In yet an embodiment, the temperature is between 2°C and 30°C, such as between 5°C and 25°C, such as between 5°C and 10°C, preferably between 5°C and 8°C.

As seen in Example 4, the strains were cultivated at 5°C, 15°C, and 25°C for measuring the SI, wherein the SI on Pikovskaya at each temperature at day 7 was:

- 2.3 at 5°C,
- 8.6 at 15°C, and
- 4.7 at 25°C.

The SI on Aleksandrow agar at each temperature at day 10 was:

- 2.2 at 5°C,
- 2.2 at 15°C, and
- 4.8 at 25°C.

In an embodiment, the bacteria or biologically pure bacterial culture is dehydrated, such as spray-dried.

In an embodiment, the bacteria or biologically pure bacterial culture does not contain the rhizoxin-biosynthetic gene cluster. In a related embodiment the bacteria or biologically pure bacterial culture does not contain the rhi and/or rzx gene loci. As explained in Example 2, this gene cluster may be toxic to human health.

In an embodiment, the bacteria or biologically pure bacterial culture further comprises one or more of a prebiotic, stabilizer, antibacterial agent, antifungal agent, preservative, and/or media component.

In a further embodiment, the prebiotic is selected from the group consisting of inulin, fructooligosaccharide, galactooligosaccharide, xylooligosaccharide, lactulose, or a combination thereof.

In a further embodiment, the stabilizer is selected from the group consisting of a sugar, a sugar alcohol, an amino acid, a lipid, or any combination thereof.

In a further embodiment, the stabilizer is selected from the group consisting of glucose, sucrose, trehalose, lactose, maltodextrin, polydextrose, dextran, fructose, oligofructose, cellulose, glycerol, adonitol, inositol, mannitol, sorbitol, gums, hydrolyzed protein, skim milk powder, milk powder, gel beads, or any combination thereof.

In an embodiment, the antibacterial agent is selected from the group consisting of bacteriocin, amoxicillin, ampicillin, azithromycin, cefaclor, cefdinir, cefotaxime, ceftazidime, ceftriaxone, cefuroxime, cephalexin, cephalosporin, ciprofloxacin, clarithromycin, clavulanate, clindamycin, clotrimazole, dalbavancin, demeclocycline, dicloxacillin, doxycycline, eravacycline, erythromycin, fluconazole, furazolidone, lansoprazole, levofloxacin, lincomycin, metronidazole, minocycline, moxifloxacin, nitroimidazole, omadacycline, oritavancin, oxacillin, penem, penicillin, penicillin V potassium, rifabutin, sulfamethoxazole, sulfasalazine, telavancin, tetracycline, tinidazole, trimethoprim, vancomycin, an antimicrobial peptide, or any combination thereof.

In yet an embodiment, the antifungal agent is amphotericin B, clotrimazole, econazole, fluconazole, itraconazole, ketoconazole, miconazole, natamycin, nystatin, posaconazole, terconazole, terbinafine, voriconazole, or any combination thereof.

In an embodiment, the bacteria or biologically pure bacterial culture according to the invention is lyophilized, spray dried, or freeze-dried.

In an embodiment, the bacteria or biologically pure bacterial culture comprises a complete biosynthetic gene cluster.

In a further embodiment, the complete biosynthetic gene cluster is a *hcnABC* gene cluster.

As seen in Example 2, DSM 34653 contains a complete biosynthetic gene cluster matching towards the *hcnABC* gene cluster encoding the secondary metabolite hydrogen cyanide, which has been suggested to act as a broad-spectrum biocontrol agent.

**Fertilizer or inoculant or biostimulant**

The isolated bacteria or biologically pure bacterial culture according to the invention may form part of different compositions. Thus, an aspect of the invention relates to a fertilizer and/or inoculant and/or biostimulant and/or biofungicide and/or antimicrobial composition comprising the bacteria or biologically pure bacterial culture according to the invention.

The composition may comprise other microorganisms. Thus, in an embodiment, the fertilizer and/or inoculant and/or biostimulant composition further comprises other microorganisms, such as other microorganisms able to function as a biostimulant.

In an embodiment, the composition further comprises one or more agriculturally acceptable carriers.

In a related embodiment, the agriculturally acceptable carrier is selected from the group consisting of a dispersant, a surfactant, an additive, water, a thickener, an anti-caking agent, residue breakdown, a composting formulation, a granular application, diatomaceous earth, an oil, a coloring agent, a stabilizer, a preservative, a polymer, biopolymer, a coating, or a combination thereof.

In yet an embodiment, the composition comprises a biopolymer, oligosaccharide, disaccharide or monosaccharide selected from the group consisting of pectin, alginate, chitosan, cellulose, a cellulose derivative, starch, maltodextrin, chitin, glucose, trehalose, sucrose, xanthan gum, guar gum, diutan gum or a biopolymer derived from a natural source, possibly chemically modified afterwards.

In another embodiment, the composition is formulated as a liquid formulation for application to plants or to a plant growth medium, or a solid formulation for application to plants or to a plant growth medium.

In yet another embodiment, the composition is formulated as a granular formulation or a powder formulation.

In an embodiment, the composition further comprises a fertilizer, a micronutrient fertilizer material, an insecticide, a herbicide, a plant growth amendment, a fungicide, a molluscicide, an algicide, a bacterial inoculant, a fungal inoculant, or a combination thereof.

In an embodiment, the composition comprises one or more of ammonium sulfate, ammonium nitrate, ammonium sulfate nitrate, ammonium chloride, ammonium bisulfate, ammonium polysulfide, ammonium thiosulfate, aqueous ammonia, anhydrous ammonia, ammonium polyphosphate, aluminum sulfate, calcium nitrate, calcium ammonium nitrate, calcium sulfate, calcined magnesite, calcitic limestone, calcium oxide, calcium nitrate, dolomitic limestone, hydrated lime, calcium carbonate, diammonium phosphate, monoammonium phosphate, magnesium nitrate, magnesium sulfate, potassium nitrate, potassium chloride, potassium magnesium sulfate, potassium sulfate, sodium nitrates, magnesian limestone, magnesia, urea, urea-formaldehydes, urea ammonium nitrate, sulfur-coated urea, polymer-coated urea, isobutylidene diurea, $K_2SO_4$-$2MgSO_4$, kainite, sylvinite, kieserite, Epsom salts, elemental sulfur, marl, ground oyster shells, fish meal, oil cakes, fish manure, blood meal, rock phosphate, super phosphates, slag, bone meal, wood ash, manure, biochar, sludge, green manure, bat guano, peat moss, compost, green sand, cottonseed meal, feather meal, crab meal, fish emulsion, or a combination thereof.

In an embodiment, the micronutrient fertilizer material comprises one or more of boric acid, a borate, a boron frit, copper sulfate, a copper frit, a copper chelate, a sodium tetraborate decahydrate, an iron sulfate, an iron oxide, iron ammonium sulfate, an iron frit, an iron chelate, a manganese sulfate, a manganese oxide, a manganese chelate, a manganese chloride, a manganese frit, a sodium molybdate, molybdic acid, a zinc sulfate, a zinc oxide, a zinc carbonate, a zinc frit, zinc phosphate, a zinc chelate, or a combination thereof.

In an embodiment, the composition is free of fungicide different from the bacteria or biologically pure bacterial culture according to the invention.

In an embodiment, the composition is formulated as a foliar spray.

In a further embodiment, the foliar spray comprises one or more of agents selected from the group consisting of tween, lecithin, saponins, plant oils, xanthan, guar gum, cellulose, carboxymethyl cellulose, agar, alginate, diutan, gellan gum, tara gum, ethoxylated fatty alcohols or ethoxylated vegetable oils, alkyl polyglucosides, organosilicones, and polyalkykeneoxide modified heptamethyltrisiloxane. The agents increase the dispersion (surfactants), adherence to the plant surface, and/or protection.

**Coating composition**

The composition according to the invention may be a coating composition, such as a seed coating composition. Thus, a further aspect of the invention relates to a coating composition, preferably a seed coating composition, comprising the bacteria or biologically pure bacterial culture according to the invention and/or the composition according to the invention.

In an embodiment, the coating composition comprises a biopolymer promoting adherence to a plant seed.

In an embodiment, the coating composition is formulated as an aqueous or oilbased solution for application to seeds, preferably aqueous.

In another embodiment, the coating composition is formulated as a powder or granular formulation for application to seeds.

In an embodiment, the coating composition is free of fungicide different from the bacteria or biologically pure bacterial culture according to the invention.

In another preferred embodiment the coating composition is in the form of a foliar spray as mentioned above. In yet an embodiment, the foliar spray comprises one or more of agents selected from the group consisting of tween, lecithin, saponins, plant oils, xanthan, guar gum, cellulose, carboxymethyl cellulose, agar, alginate, diutan, gellan gum, tara gum, ethoxylated fatty alcohols or ethoxylated vegetable oils, alkyl polyglucosides, organosilicones, and polyalkykeneoxide modified heptamethyltrisiloxane.

In an embodiment, the coating composition further comprises one or more agriculturally acceptable carriers.

In a related embodiment, the agriculturally acceptable carrier is selected from the group consisting of a dispersant, a surfactant, an additive, water, a thickener, an anti-caking agent, residue breakdown, a composting formulation, a granular application, diatomaceous earth, an oil, a coloring agent, a stabilizer, a preservative, a polymer, biopolymer, a coating, or a combination thereof.

In yet an embodiment, the coating composition comprises a biopolymer, oligosaccharide, disaccharide or monosaccharide selected from the group consisting of pectin, alginate, chitosan, cellulose, a cellulose derivative, starch, maltodextrin, chitin, glucose, trehalose, sucrose, xanthan gum, guar gum, diutan gum or a biopolymer derived from a natural source, possibly chemically modified afterwards.

It is to be understood that embodiments mentioned for one aspect of the invention may also be combinable with other aspects of the invention. For example, embodiments described under "Fertilizer or inoculant or biostimulant" may be combined with aspects under coating compositions.

## Coated plant seed

The present invention also relates to seeds coated with the compositions according to the invention. Thus, an aspect relates to a plant seed coated with the composition according to the invention or coated with a coating composition according to the invention.

In an embodiment, the plant seed is a dicotyledon, monocotyledon or a gymnosperm seed.

In yet an embodiment, the plant seed being selected from the group consisting of a crop seed, such as barley seed, such as spring or winter barley, oilseed, such as rapeseed or oil radish seeds, wheat, such as winter or spring wheat, oats, triticale, maize, rye, grass, clover, broad bean, lupines, strawberries, tomatoes, cucumber, peas, potatoes, onions, carrots and sugar beets.

As seen in Example 5, spring barley seeds coated with the composition according to the invention or coated with a coating composition according to the invention are shown to result in an enhanced emergence of seedlings, thus increased growth and yield.

In yet an embodiment, the plant seed being selected from the group consisting of a cover crop seed, such as fodder radish, clover, yellow mustard or *Phacelia*.

In yet an embodiment, the plant seed being selected from the group consisting of trees, bushes or grasses.

In an embodiment, the plant seed is free of fungicide different from the bacteria or biologically pure bacterial culture according to the invention.

## Coated plant leaves

Similar to coating plant seeds, the present disclosure also relates to coated plant leaves. Thus, yet an aspect of the disclosure relates to plant leaves coated with the composition according to the invention or coated with a coating composition according to the invention (such as a foliar spray).

## Uses and methods

An aspect of the invention relates to the use of an (isolated) bacteria or biologically pure bacterial culture according to the invention, the composition according to the invention or the coating composition according to the invention, as a plant growth promoting agent, such as a fertilizer or inoculum or biostimulant, preferably at a temperature below 15°C, such as below 10°C, preferably between 2°C and 8°C, more preferably around 5°C.

In an embodiment, the temperature is a surface temperature.

In an embodiment, the use as a plant growth-promoting agent is for:

- solubilizing inorganic minerals or salts, such as phosphate, and/or potassium, and/or iron, and/or aluminium, and/or zinc, and/or manganese; and/or
- chelating inorganic minerals, such as iron, zinc, manganese, copper; and/or
- allowing for growth under low soil nitrogen conditions; and/or
- fixating atmospheric nitrogen; and/or
- increasing plant growth, such as plant length, leaf diameter and/or root length; and/or
- improving germination of the seeds; and/or
- improving emergence of the plants; and/or
- increasing biomass; and/or
- increasing growth; and/or
- increasing crop yield; and/or
- inducing anti-fungal effects; and/or
- increasing harvest yield; and/or
- increasing hectoliter weight; and/or
- increasing protein content; and/or
- increasing protein yield; and/or
- increasing oil content; and/or
- increasing oil yield; and/or
- reducing mycotoxin contamination; and/or
- reducing seed-borne fungal or bacterial contamination; and/or
- improving taste, smell or appearance; and/or
- replacing or substituting any chemical product used in plant production while retaining one or more desirable plant parameters.

The stimulation of plant growth achieved by the present methods and uses can be measured in a number of ways. Stimulation of plant growth can be determined by increase in the average **height of the plant,** such as an increase of at least 5%, by at least 10%, by at least 15% or by at least 20% as compared to the average height of plants grown under the same conditions but that have not been treated according to the present invention. Also, stimulation of plant growth can be determined by an increase in the **average leaf diameter** of the leaves of plant, such as an increase of at least 5%, of at least 10%, of at least 15% or of at least 20% as compared to the average leaf diameter of plants grown under the same conditions but that have not been treated according to the present invention. Similarly, stimulation of plant growth can be shown by an increase in instances the average **root length** of the plant, such as an increase of at least 5%, of at least 10%, of at least 15% or of at least 20% as compared to the average root length of the plants grown under the same conditions but that have not been treated according to the present invention. As outlined above, stimulation of plant growth can also be estimated using other parameters.

In an embodiment, the inorganic minerals or salts solubilized are selected from the group consisting of

- minerals, such as rock phosphate, potash, lime, clay, ground rocks, sand, silt, sediment and natural deposits;
- salts, such as ammonium phosphate, potassium phosphate, potassium nitrate, potassium chloride, ammonium sulfate, calcium phosphate; calcium sulphate, magnesium phosphate, iron phosphate, potassium alumino silicate (feldspar, mica), and salts that contain potassium or phosphate
- fertilizing substances, such as mineral fertilizer, NPK fertilizer, NS fertilizer, K fertilizer, P fertilizer, N fertilizer, organic fertilizer, manure, sludge, compost, biowaste, biochar, biogas residue, ash, wood ash, bone ash, bone meal, urine, faeces or a plant-based fertilizer.

The isolated bacteria or biologically pure bacterial culture can grow under low temperature conditions. Thus, in an embodiment, the use takes place at field temperatures (measured as the soil surface temperature) in the range -10°C to 15°C, such as -5°C to 15°C, preferably 0 to 15°C, more preferably such as 2 to 10°C, such 2 to 8°C.

In another embodiment, the (isolated) bacteria or biologically pure bacterial culture according to the invention, the composition according to the invention or the coating composition according to the invention is applied in an effective amount.

In yet another embodiment, the use takes place in Scandinavia, such as Norway, Sweden, Finland, Denmark, such as in Jutland, Fyn, Zealand, and/or Great Britain, and /or Germany.

In an embodiment, the plant is a dicotyledon, monocotyledon or a gymnosperm.

In another embodiment, the plant is selected from the group consisting of a crop seed, such as barley seed, such as spring barley, oilseed, such as rapeseed, wheat, such as winter wheat.

**The dicotyledon** can be selected from the group consisting of bean, pea, tomato, pepper, squash, alfalfa, almond, aniseed, apple, apricot, arracha, artichoke, avocado, bambara groundnut, beet, bergamot, black pepper, black wattle, blackberry, blueberry, bitter orange, bok- choi, Brazil nut, breadfruit, broccoli, broad bean, Brussels sprouts, buckwheat, cabbage, camelina, Chinese cabbage, cacao, cantaloupe, caraway seeds, cardoon, carob, carrot, cashew nuts, cassava, castor bean, cauliflower, celeriac, celery, cherry, chestnut, chickpea, chicory, chili pepper, chrysanthemum, cinnamon, citron, Clementine, clove, clover, coffee, cola nut, colza, corn, cotton, cottonseed, cowpea, crambe, cranberry, cress, cucumber, currant, custard apple, drumstick tree, earth pea, eggplant, endive, fennel, fenugreek, fig, filbert, flax, geranium, gooseberry, gourd, grape, grapefruit, guava, hemp, hempseed, henna, hop, horse bean, horseradish, indigo, jasmine, Jerusalem artichoke, jute, kale, kapok, kenaf, kohlrabi, kumquat, lavender, lemon, lentil, lespedeza, lettuce, lime, liquorice, litchi, loquat, lupine, macadamia nut, mace, mandarin, mangel, mango, medlar, melon, mint, mulberry, mustard, nectarine, niger seed, nutmeg, okra, olive, opium, orange, papaya, parsnip, pea, peach, peanut, pear, pecan nut, persimmon, pigeon pea, pistachio nut, plantain, plum, pomegranate, pomelo, poppy seed, potato, sweet potato, prune, pumpkin, quebracho, quince, trees of the genus Cinchona, quinoa, radish, ramie, rapeseed, raspberry, rhea, rhubarb, rose, rubber, rutabaga, safflower, sainfoin, salsify, sapodilla, Satsuma, scorzonera, sesame, shea tree, soybean, spinach, squash, strawberry, sugar beet, sugarcane, sunflower, swede, sweet pepper, tangerine, tea, teff, tobacco, tomato, trefoil, tung tree, turnip, urena, vetch, walnut, watermelon, yerba mate, wintercress, shepherd's purse, garden cress, peppercress, watercress, pennycress, star anise, laurel, bay laurel, cassia, jamun, dill, tamarind, peppermint, oregano, rosemary, sage, soursop, pennywort, calophyllum, balsam pear, kukui nut, Tahitian chestnut, basil, huckleberry, hibiscus, passionfruit, star apple, sassafras, cactus, St. John's wort, loosestrife, hawthorn, cilantro, curry plant, kiwi, thyme, zucchini, ulluco, jicama, waterleaf, spiny monkey orange, yellow mombin, starfruit, amaranth, wasabi, Japanese pepper, yellow plum, mashua, Chinese toon, New Zealand spinach, bower spinach, ugu, tansy, chickweed, jocote, Malay apple, paracress, sowthistle, Chinese potato, horse parsley, hedge mustard, campion, agate, cassod tree, thistle, burnet, star gooseberry, saltwort, glasswort, sorrel, silver lace fern, collard greens, primrose, cowslip, purslane, knotgrass, terebinth, tree lettuce, wild betel, West African pepper, yerba santa, tarragon, parsley, chervil, land cress, burnet saxifrage, honeyherb, butterbur, shiso, water pepper, perilla, bitter bean, oca, kampong, Chinese celery, lemon basil, Thai basil, water mimosa, cicely, cabbage-tree, moringa, mauka, ostrich fern, rice paddy herb, yellow sawah lettuce, lovage, pepper grass, maca, bottle gourd, hyacinth bean, water spinach, catsear, fishwort, Okinawan spinach, lotus sweetjuice, gallant soldier, culantro, arugula, cardoon, caigua, mitsuba, chipilin, samphire, mampat, ebolo, ivy gourd, cabbage thistle, sea kale, chaya, huauzontle, Ethiopian mustard, magenta spreen, good king henry, epazole, lamb's quarters, centella plumed cockscomb, caper, rapini, napa cabbage, mizuna, Chinese savoy, kai-lan, mustard greens, Malabar spinach, chard, marshmallow, climbing wattle, China jute, paprika, annatto seed, spearmint, savory, marjoram, cumin, chamomile, lemon balm, allspice, bilberry, cherimoya, cloudberry, damson, pitaya, durian, elderberry, feijoa, jackfruit, jambul, jujube, physalis, purple mangosteen, rambutan, redcurrant, blackcurrant, salal berry, satsuma, ugli fruit, azuki bean, black bean, black-eyed pea, borlotti bean, common bean, green bean, kidney bean, lima bean, mung bean, navy bean, pinto bean, runner bean, mangetout, snap pea, broccoflower, calabrese, nettle, bell pepper, raddichio, daikon, white radish, skirret, tat soi, broccolini, black radish, burdock root, fava bean, broccoli raab, lablab, lupin, sterculia, velvet beans, winged beans, yam beans, mulga, ironweed, umbrella bush, tjuntjula, wakalpulka, witchetty bush, wiry wattle, chia, beech nut, candlenut, colocynth, mamoncillo, Maya nut, mongongo, ogbono nut, paradise nut, and cempedak.

**The dicotyledon** can be from a family selected from the group consisting of Acanthaceae (acanthus), Aceraceae (maple), Achariaceae, Achatocarpaceae (achatocarpus), Actinidiaceae (Chinese gooseberry), Adoxaceae (moschatel), Aextoxicaceae, Aizoaceae (fig marigold), Akaniaceae, Alangiaceae, Alseuosmiaceae, Alzateaceae, Amaranthaceae (amaranth), Amborellaceae, Anacardiaceae (sumac), Ancistrocladaceae, Anisophylleaceae, Annonaceae (custard apple), Apiaceae (carrot), Apocynaceae (dogbane), Aquifoliaceae (holly), Araliaceae (ginseng), Aristolochiaceae (birthwort), Asclepiadaceae (milkweed), Asteraceae (aster), Austrobaileyaceae, Balanopaceae, Balanophoraceae (balanophora), Balsaminaceae (touch-me- not), Barbeyaceae, Barclayaceae, Basellaceae (basella), Bataceae (saltwort), Begoniaceae (begonia), Berberidaceae (barberry), Betulaceae (birch), Bignoniaceae (trumpet creeper), Bixaceae (lipstick tree), Bombacaceae (kapok tree), Boraginaceae (borage), Brassicaceae (mustard, also Cruciferae), Bretschneideraceae, Brunelliaceae (brunellia), Bruniaceae, Brunoniaceae, Buddlejaceae (butterfly bush), Burseraceae (frankincense), Buxaceae (boxwood), Byblidaceae, Cabombaceae (water shield), Cactaceae (cactus), Caesalpiniaceae, Callitrichaceae (water starwort), Calycanthaceae (strawberry shrub), Calyceraceae (calycera), Campanulaceae (bellflower), Canellaceae (canella), Cannabaceae (hemp), Capparaceae (caper), Caprifoliaceae (honeysuckle), Cardiopteridaceae, Caricaceae (papaya), Caryocaraceae (souari), Caryophyllaceae (pink), Casuarinaceae (she-oak), Cecropiaceae (cecropia), Celastraceae (bittersweet), Cephalotaceae, Ceratophyllaceae (hornwort), Cercidiphyllaceae (katsura tree), Chenopodiaceae (goosefoot), Chloranthaceae (chloranthus), Chrysobalanaceae (cocoa plum), Circaeasteraceae, Cistaceae (rockrose), Clethraceae (clethra), Clusiaceae (mangosteen, also Guttiferae), Cneoraceae, Columelliaceae, Combretaceae (Indian almond), Compositae (aster), Connaraceae (cannarus), Convolvulaceae (morning glory), Coriariaceae, Cornaceae (dogwood), Corynocarpaceae (karaka), Crassulaceae (stonecrop), Crossosomataceae (crossosoma), Crypteroniaceae, Cucurbitaceae (cucumber), Cunoniaceae (cunonia), Cuscutaceae (dodder), Cyrillaceae (cyrilla), Daphniphyllaceae, Datiscaceae (datisca), Davidsoniaceae, Degeneriaceae, Dialypetalanthaceae, Diapensiaceae (diapensia), Dichapetalaceae, Didiereaceae, Didymelaceae, Dilleniaceae (dillenia), Dioncophyllaceae, Dipentodontaceae, Dipsacaceae (teasel), Dipterocarpaceae (meranti), Donatiaceae, Droseraceae (sundew), Duckeodendraceae, Ebenaceae (ebony), Elaeagnaceae (oleaster), Elaeocarpaceae (elaeocarpus), Elatinaceae (waterwort), Empetraceae (crowberry), Epacridaceae (epacris), Eremolepidaceae (catkin-mistletoe), Ericaceae (heath), Erythroxylaceae (coca), Eucommiaceae, Eucryphiaceae, Euphorbiaceae (spurge), Eupomatiaceae, Eupteleaceae, Fabaceae (pea or legume), Fagaceae (beech), Flacourtiaceae (flacourtia), Fouquieriaceae (ocotillo), Frankeniaceae (frankenia), Fumariaceae (fumitory), Garryaceae (silk tassel), Geissolomataceae, Gentianaceae (gentian), Geraniaceae (geranium), Gesneriaceae (gesneriad), Globulariaceae, Gomortegaceae, Goodeniaceae (goodenia), Greyiaceae, Grossulariaceae (currant), Grubbiaceae, Gunneraceae (gunnera), Gyrostemonaceae, Haloragaceae (water milfoil), Hamamelidaceae (witch hazel), Hernandiaceae (hernandia), Himantandraceae, Hippocastanaceae (horse chestnut), Hippocrateaceae (hippocratea), Hippuridaceae (mare's tail), Hoplestigmataceae, Huaceae, Hugoniaceae, Humiriaceae, Hydnoraceae, Hydrangeaceae (hydrangea), Hydrophyllaceae (waterleaf), Hydrostachyaceae, Icacinaceae (icacina), Idiospermaceae, Illiciaceae (star anise), Ixonanthaceae, Juglandaceae (walnut), Julianiaceae, Krameriaceae (krameria), Lacistemataceae, Lamiaceae (mint, also Labiatae), Lardizabalaceae (lardizabala), Lauraceae (laurel), Lecythidaceae (brazil nut), Leeaceae, Leitneriaceae (corkwood), Lennoaceae (lennoa), Lentibulariaceae (bladderwort), Limnanthaceae (meadow foam), Linaceae (flax), Lissocarpaceae, Loasaceae (loasa), Loganiaceae (logania), Loranthaceae (showy mistletoe), Lythraceae (loosestrife), Magnoliaceae (magnolia), Malesherbiaceae, Malpighiaceae (barbados cherry), Malvaceae (mallow), Marcgraviaceae (shingle plant), Medusagynaceae, Medusandraceae, Melastomataceae (melastome), Meliaceae (mahogany), Melianthaceae, Mendonciaceae, Menispermaceae (moonseed), Menyanthaceae (buckbean), Mimosaceae, Misodendraceae, Mitrastemonaceae, Molluginaceae (carpetweed), Monimiaceae (monimia), Monotropaceae (Indian pipe), Moraceae (mulberry), Moringaceae (horseradish tree), Myoporaceae (myoporum), Myricaceae (bayberry), Myristicaceae (nutmeg), Myrothamnaceae, Myrsinaceae (myrsine), Myrtaceae (myrtle), Nelumbonaceae (lotus lily), Nepenthaceae (East Indian pitcherplant), Neuradaceae, Nolanaceae, Nothofagaceae, Nyctaginaceae (four-o'clock), Nymphaeaceae (water lily), Nyssaceae (sour gum), Ochnaceae (ochna), Olacaceae (olax), Oleaceae (olive), Oliniaceae, Onagraceae (evening primrose), Oncothecaceae, Opiliaceae, Orobanchaceae (broom rape), Oxalidaceae (wood sorrel), Paeoniaceae (peony), Pandaceae, Papaveraceae (poppy), Papilionaceae, Paracryphiaceae, Passifloraceae (passionflower), Pedaliaceae (sesame), Pellicieraceae, Penaeaceae, Pentaphragmataceae, Pentaphylacaceae, Peridiscaceae, Physenaceae, Phytolaccaceae (pokeweed), Piperaceae (pepper), Pittosporaceae (pittosporum), Plantaginaceae (plantain), Platanaceae (plane tree), Plumbaginaceae (leadwort), Podostemaceae (river weed), Polemoniaceae (phlox), Polygalaceae (milkwort), Polygonaceae (buckwheat), Portulacaceae (purslane), Primulaceae (primrose), Proteaceae (protea), Punicaceae (pomegranate), Pyrolaceae (shinleaf), Quiinaceae, Rafflesiaceae (rafflesia), Ranunculaceae (buttercup orranunculus), Resedaceae (mignonette), Retziaceae, Rhabdodendraceae, Rhamnaceae (buckthorn), Rhizophoraceae (red mangrove), Rhoipteleaceae, Rhynchocalycaceae, Rosaceae (rose), Rubiaceae (madder), Rutaceae (rue), Sabiaceae (sabia), Saccifoliaceae, Salicaceae (willow), Salvadoraceae, Santalaceae (sandalwood), Sapindaceae (soapberry), Sapotaceae (sapodilla), Sarcolaenaceae, Sargentodoxaceae, Sarraceniaceae (pitcher plant), Saururaceae (lizard's tail), Saxifragaceae (saxifrage), Schisandraceae (schisandra), Scrophulariaceae (figwort), Scyphostegiaceae, Scytopetalaceae, Simaroubaceae (quassia), Simmondsiaceae (jojoba), Solanaceae (potato), Sonneratiaceae (sonneratia), Sphaerosepalaceae, Sphenocleaceae (spenoclea), Stackhousiaceae (stackhousia), Stachyuraceae, Staphyleaceae (bladdernut), Sterculiaceae (cacao), Stylidiaceae, Styracaceae (storax), Surianaceae (suriana), Symplocaceae (sweetleaf), Tamaricaceae (tamarix), Tepuianthaceae, Tetracentraceae, Tetrameristaceae, Theaceae (tea), Theligonaceae, Theophrastaceae (theophrasta), Thymelaeaceae (mezereum), Ticodendraceae, Tiliaceae (linden), Tovariaceae, Trapaceae (water chestnut), Tremandraceae, Trigoniaceae, Trimeniaceae, Trochodendraceae, Tropaeolaceae (nasturtium), Turneraceae (turnera), Ulmaceae (elm), Urticaceae (nettle), Valerianaceae (valerian), Verbenaceae (verbena), Violaceae (violet), Viscaceae (Christmas mistletoe), Vitaceae (grape), Vochysiaceae, Winteraceae (wintera), Xanthophyllaceae, and Zygophyllaceae (creosote bush).

**The monocotyledon** can be selected from the group consisting of corn, wheat, oat, rice, barley, millet, banana, onion, garlic, asparagus, ryegrass, millet, fonio, raishan, nipa grass, turmeric, saffron, galangal, chive, cardamom, date palm, pineapple, shallot, leek, scallion, water chestnut, ramp, Job's tears, bamboo, ragi, spotless watermeal, arrowleaf elephant ear, Tahitian spinach, abaca, areca, bajra, betel nut, broom millet, broom sorghum, citronella, coconut, cocoyam, maize, dasheen, durra, durum wheat, edo, fique, formio, ginger, orchard grass, esparto grass, Sudan grass, guinea corn, Manila hemp, henequen, hybrid maize, jowar, lemon grass, maguey, bulrush millet, finger millet, foxtail millet, Japanese millet, proso millet, New Zealand flax, oats, oil palm, palm palmyra, sago palm, redtop, sisal, sorghum, spelt wheat, sweet corn, sweet sorghum, taro, teff, timothy grass, triticale, vanilla, wheat, and yam.

**Alternatively, the monocotyledon** can be selected from a family selected from the group consisting of Acoraceae (calamus), Agavaceae (century plant), Alismataceae (water plantain), Aloeaceae (aloe), Aponogetonaceae (cape pondweed), Araceae (arum), Arecaceae (palm), Bromeliaceae (bromeliad), Burmanniaceae (burmannia), Butomaceae (flowering rush), Cannaceae (canna), Centrolepidaceae, Commelinaceae (spiderwort), Corsiaceae, Costaceae (costus), Cyanastraceae, Cyclanthaceae (Panama hat), Cymodoceaceae (manatee grass), Cyperaceae (sedge), Dioscoreaceae (yam), Eriocaulaceae (pipewort), Flagellariaceae, Geosiridaceae, Haemodoraceae (bloodwort), Hanguanaceae (hanguana), Heliconiaceae (heliconia), Hydatellaceae, Hydrocharitaceae (tape grass), Iridaceae (iris), Joinvilleaceae (joinvillea), Juncaceae (rush), Juncaginaceae (arrow grass), Lemnaceae (duckweed), Liliaceae (lily), Limnocharitaceae (water poppy), Lowiaceae, Marantaceae (prayer plant), Mayacaceae (mayaca), Musaceae (banana), Najadaceae (water nymph), Orchidaceae (orchid), Pandanaceae (screw pine), Petrosaviaceae, Philydraceae (philydraceae), Poaceae (grass), Pontederiaceae (water hyacinth), Posidoniaceae (posidonia), Potamogetonaceae (pondweed), Rapateaceae, Restionaceae, Ruppiaceae (ditch grass), Scheuchzeriaceae (scheuchzeria), Smilacaceae (catbrier), Sparganiaceae (bur reed), Stemonaceae (stemona), Strelitziaceae, Taccaceae (tacca), Thurniaceae, Triuridaceae, Typhaceae (cattail), Velloziaceae, Xanthorrhoeaceae,, Xyridaceae (yellow-eyed grass), Zannichelliaceae (horned pondweed), Zingiberaceae (ginger), and Zosteraceae (eelgrass).

**The gymnosperm** can be selected from a family selected from the group consisting of Araucariaceae, Boweniaceae, Cephalotaxaceae, Cupressaceae, Cycadaceae, Ephedraceae, Ginkgoaceae, Gnetaceae, Pinaceae, Podocarpaceae, Taxaceae, Taxodiaceae, Welwitschiaceae, and Zamiaceae.

## Method for stimulating plant growth

In a further aspect, the invention relates to a method for stimulating plant growth comprising applying the (isolated) bacteria or biologically pure bacterial culture according to the invention, the composition according to the invention or the coating composition according to the invention to a plant, plant seed, a sowing furrow, soil and/or plant growth medium, preferably at a temperature below 15°C, such as below 10°C, preferably between 2°C and 8°C, more preferably around 5°C.

In an embodiment, the method comprises applying the bacteria or biologically pure bacterial culture according to the invention, the composition according to the invention or the coating composition according to the invention:

- to a plant growth medium, such as sphagnum; and/or

- to a plant growth medium prior to, concurrently with, or after planting of seeds, seedlings, cuttings, bulbs, or plants in the plant growth medium; or
- to plant leaves, roots, or stems; and/or
- to plant seeds, and/or
- to a watering system for the plants, such as hydroponics, aeroponics and/or aquaponics.

In yet an embodiment, the bacteria or biologically pure bacterial culture according to the invention, the composition according to the invention or the coating composition according to the invention is sprayed or irrigated onto plants or fields.

**Kit**

In yet a further aspect, the invention relates to a kit of parts for stimulating plant growth comprising

- a first container comprising the (isolated) bacteria or biologically pure bacterial culture according to the invention, the composition according to the invention and/or the coating composition according to the invention; and
- instructions for applying the (isolated) bacteria or biologically pure bacterial culture according to the invention, the composition according to the invention and/or the coating composition according to the invention to plants, plant seeds, or a plant growth medium, preferably at a temperature below 15°C, such as below 10°C, preferably between 2°C and 8°C, more preferably around 5°C.

In an embodiment, the kit of parts further comprises one or more containers comprising fertilizers, nutrients, and/or other microorganisms.

**Ores**

A further aspect of the disclosure relates to use of the (isolated) bacteria or biologically pure bacterial culture according to the invention or the composition according to the invention for solubilizing minerals in ores.
The bacteria may be added to an ore containing e.g. insoluble calcium phosphate, dissolving the mineral and releasing soluble calcium and phosphate that may be extracted from the ore. This process is also known as bioleaching and biomining.

**Medical uses**

A yet an aspect of the present invention relates to a bacteria or biologically pure bacterial culture according to the invention, a composition according to the invention or a coating composition according to the invention for use as a medicament.
Another aspect relates to a bacteria or biologically pure bacterial culture according to the invention or a composition according to the invention use in the treatment, alleviation and/or prevention of fungal infections.
In a further embodiment, the fungal infection is selected from the group consisting of *Gaeumannomyces graminis var. tritici, Pyrenophora teres f. teres, Zymoseptoria tritici, Fusarium oxysporum* or a combination thereof.
As can be seen in Example 3, the bacteria or biologically pure bacterial culture according to the invention comprises antifungal activity.
It should be noted that embodiments and features described in the context of one of the aspects of the present invention also apply to the other aspects of the invention.
The invention will now be described in further details in the following non-limiting examples.

**Examples**

**Example 1** - **Isolation of bacterial strains from a Danish grassland with biocontrol properties**

*Aim of study*

The scope of this example is to demonstrate how bacterial strains with biocontrol properties were isolated from the soil of a Danish grassland and screened to identify the best-performing bacterial strain.

*Materials and methods*

The microbeTRAP [WO2021/180941 A1] was 3D printed from PA (SLS, Materialize), it contained 32 paired chambers, where 16 of the chambers contained a nutrient pill and the other 16 a capture pill. Each of the nutrient pills were connected to a separate chamber containing a capture pill.

A microbeTRAPs were prepared to select for microorganisms with biocontrol properties: prior to incubation the capture pills were soaked in a LB broth containing *Saccharomyces cerevisiae (S. cerevisiae)* for 24 hours to ensure cultivation of this organism within the capture pill. Nutrient pills and capture pills were then placed in each microbeTRAP and it was then sealed with a lid. Soil from an old grassland on southern Funen, Denmark (coordinates: 55°02'34.8"N 10°24'43.3"E) was collected in December 2021. The soil was placed in a biosafety plastic box indoor to ensure a temperature of approximately 20°C during incubation. The microbeTRAP was placed in the middle of the box and covered with around 10 cm of soil. The soil was moistened with 1 L of sterile water during the incubation period to create a moist environment that would promote nutrient exchange and chemotaxi. The microbeTRAPs were excavated after 14 days and the capture pills retrieved. Capture pills were cultured on LB agar plates covered with S. cerevisiae (100 µL, 5 McF solution). The plates were incubated at 20°C for 2 days. Capture pills with clearing zones of S. *cerevisiae* were sampled for microorganisms using an inoculation loop that were swept from the capture pill out to the edge of the colony (n = 12). The microorganisms were purified on LB agar using the 4-streak method. This step was repeated until the colonies were pure and only contained one bacterial strain. Pure cultures were transferred to new LB plates covered with *S. cerevisiae* to confirm inhibition was retained. Inhibition was confirmed in 7 of the 12 strains.

A dual culture assay was used to screen for the most efficient biocontrol candidate among the strains (n = 7). Three phytopathogenic fungi were used in the assay: *Bipolaris maydis* (isolate CP2050), *Pyrenophora teres f. teres* (isolate CP2189), and *Zymoseptoria tritici* (isolate IPO323). A spot on lawn setup was employed: petri dishes containing different nutrient agar were covered with a solution of fungal spores. Subsequently a solution of bacteria was spotted onto the plate in three droplets. A solution of fungal spores was prepared in 1:10 PBS and 100 µL were spread on nutrient agar. *Bipolaris maydis* were cultivated on 1/3 strength potato dextrose agar (PDA) and regular PDA, *Pyrenophora teres f. teres* was cultivated on grass agar and PDA, and *Zymoseptoria tritici* was cultivated on PDA. Solutions of bacterial strains were prepared in a concentration of 0.5 McF in 1:10 PBS and 10 µL was spotted onto the plates in replicates of three. The plates were incubated at 20°C for 7 days. Inhibition was semi-qualitatively evaluated by scoring the degree of inhibition from 0-4, where 0 indicates no inhibition and 1-4 indicates increasing inhibition zones around the bacterial colonies.

*Results*

From the initial screening of capture pills on plates covered with *S. cerevisiae* 12 of 32 capture pills were identified with clearing zones around them. In the following screening of purified cultures 7 strains (TF-A, TF-B. TF-C, TF-D, TF-E, TF-F, TF-G) were identified to maintain this capacity. These 7 strains were used dual culture screening test with 3 common phytopathogenic fungi **(Table 1).** All strains inhibited growth of all fungi on at least one of the tested nutrient agars. TF-B, TF-D, and TF-G had the highest total inhibition score. As TF-D was the only strain inhibiting growth of *Pyrenophora teres f. teres* on grass, and furthermore exhibited largely similar inhibition score as the other two, this strain was selected for further analysis. TF-D was deposited at DSMZ with the accession number DSM 34653 and the strain was from here on referred to as DSM 34653.

**Table 1:** *Fungal inhibition by the 7 bacterial strains isolated from the MicrobeTRAP. Three phytopathogenic fungi were tested Bipolaris maydis (isolate CP2050), Pyrenophora teres f. teres (isolate CP2189), and Zymoseptoria tritici (isolate IPO323). The fungal inhibition was scored from 0-4, where 0 indicates no inhibition and 1-4 indicates increasing inhibition zones around tested bacterial strains.*

| | Fungal inhibition | | | | | Total score |
|---|---|---|---|---|---|---|
| | **B. maydis** | | **P. teres** | | **Z. tritici** | |
| | 1/3 PDA | PDA | Grass | PDA | PDA | |
| | Day 7 | Day 7 | Day 7 | Day 7 | Day 7 | |
| Negative control | 0 | 0 | 0 | 0 | 0 | 0 |
| TF-A | 1 | 1 | 0 | 2 | 1 | 5 |
| TF-B | 1 | 2 | 0 | 2 | 2 | 7 |
| TF-C | 2 | 0 | 0 | 3 | 1 | 6 |
| **TF-D** | **2** | **0** | **1** | **2** | **3** | **8** |

(continued)

| | Fungal inhibition | | | | | Total score |
|---|---|---|---|---|---|---|
| | **B. maydis** | | **P. teres** | | **Z. tritici** | |
| | 1/3 PDA | PDA | Grass | PDA | PDA | |
| | Day 7 | Day 7 | Day 7 | Day 7 | Day 7 | |
| TF-E | 3 | 1 | 0 | 2 | 1 | 7 |
| TF-F | 2 | 0 | 0 | 2 | 1 | 5 |
| TF-G | 1 | 3 | 0 | 2 | 3 | 9 |

*Conclusion*

The microbeTRAP enabled capture of multiple potential bacterial biocontrol strains from Danish grassland soil. Of the isolated strains, TF-D (DSM 34653) was chosen as the most promising candidate for a biocontrol agent based on the initial screening for biocontrol properties on three common phytopathogenic fungi.

**Example 2** - **Characterizing DSM 34653 as a novel** *Pseudomonas* **species**

*Aim of study*

The scope of this example is to demonstrate that DSM 34653, which was identified as the best-performing biocontrol strain from a screening setup (TF-D in **Example 1),** is a novel bacterial species belonging to the genus *Pseudomonas*.

*Materials and methods*

*Illumina short-read DNA sequencing*

DNA was extracted from a bacterial culture of DSM 34653, using the DNeasy UltraClean Microbial Kit from Qiagen following the instructions from the manufacturer. 50 ng of DNA was prepared for Illumina paired-end sequencing (2x150 bp reads) using the TWIST Library Preparation EF 2.0 enzymatic fragmentation kit following the instructions provided by the manufacturer. The DNA library was sequenced on an Illumina MiniSeq machine. A total of 5,950,818 reads were obtained.

*ONT long-read DNA sequencing*

DNA was extracted from a bacterial culture of DSM 34653 using the DNeasy UltraClean Microbial Kit from Qiagen following the instructions from the manufacturer. 400 ng of DNA was prepared for Oxford Nanopore Technologies (ONT) whole genome sequencing using the Native Barcoding Kit 24 V14 (SQK-NBD114.24). The DNA library was sequenced on a MinION Mk1b device using a R10.4.1 flow cell until a sequencing depth of minimum 100x was obtained.

*Quality control and processing of Illumina data*

Adapter and barcode contaminants originating from library preparation steps were removed from the obtained sequencing data using Trim Galore (v0.6.7) while retaining nucleotides with a Phred score >20. Only quality-controlled reads were considered for downstream analyses. A total of 5,950,066 reads were retained.

*Quality control and processing of ONT data*

Adapter and barcode contaminants originating from library preparation steps were removed while basecalling raw ONT data with the super accuracy (SUP) model using Guppy (v6.4.6). To obtain ONT data with high accuracy, duplex basecalling was subsequently performed using Guppy (v6.4.6) and Duplex Tools (v0.2.17) which utilizes both complement and template DNA strands to generate consensus basecalled data with a high confidence (9.2% of all data was identified as good read pairs). Combining duplex data with the SUP basecalled ONT data (where only the template DNA strand was basecalled), high-accuracy and high-coverage ONT data was obtained. To ensure that only high-quality reads were kept, chopper (v0.2.0) was used for retaining sequencing reads with an average Phred score >20 and a read length of minimum 500 bp. Only quality-controlled reads were considered for downstream analyses corresponding to a total of 512,878,87 bp.

*Taxonomic classification of sequencing reads*

Kraken2 (v2.1.2) was used for assigning Illumina and ONT sequencing data according to the National Center for Biotechnology Information (NCBI) Taxonomy. A comprehensive pre-built database containing complete (as of October 09, 2023, downloaded from https://benlangmead.github.io/aws-indexes/k2) RefSeq genomes mapping to archea, bacteria, viruses, plasmids, humans, fungi, protozoans as well as vector sequences was used as a reference for the taxonomic assignment. Bracken (v2.8) was subsequently used for abundance re-estimation at genus and species level.

*Hybrid de novo genome assembly and assembly statistics*

A complete de novo genome for DSM 34653 was obtained through a hybrid assembly approach utilizing both short Illumina and long ONT quality-controlled reads. The long ONT reads were used for generating a non-fragmented de novo genome using Flye (v2.9.2) and the short Illumina reads were subsequently used for error-correction at the nucleotide level using Pilon (v1.24). QUAST (v5.0.2) was used for computing assembly quality metrics.

*Genome annotation*

Genomic features present in the assembled chromosome were annotated using Prokka (v1.14.6).

*Average nucleotide identity (ANI) analysis*

FastANI (v1.34) was used for computing average nucleotide identity (ANI) scores of the assembled genome of DSM 34653 compared to all *Pseudomonas* RefSeq reference genomes available at NCBI (n=368 excluding atypical genomes, as of April 3, 2024).

*Whole genome-based phylogenetic reconstruction*

SANS ambages (v2.3_9A) was used for determining genome-to-genome evolutionary relationships of DSM 34653 and all *Pseudomonas* RefSeq reference genomes available at NCBI (n=368, excluding atypical genomes, as of April 3 2024) or all *P. wadenswilerensis, P. donghuensis, P. tructae* and *P. rubra* RefSeq strain genomes available at NCBI (n=14, excluding atypical genomes, as of April 5, 2024). The computed phylogenetic splits were subsequently visualized as phylogenetic trees using R (v4.3.3) and ggtree (v3.8.2).

*Secondary metabolite analysis*

antiSMASH (v7.0.0) was used for the identification and annotation of secondary metabolite biosynthesis gene clusters within the genome assembly of DSM 34653. The Minimum Information about a Biosynthetic Gene cluster (MIBiG) repository containing experimentally characterized biosynthetic gene clusters was used for annotation.

*Virulence factor analysis*

RefSeq genomes of four *P. aeruginosa* strains (PAO1, UCBPP PA14, LESB58 and PA7) were downloaded from NCBI (on April 16, 2024) and annotated using Prokka (see the *Genome annotation* section). Translated nucleotide sequences corresponding to genes identified by Prokka from each *P. aeruginosa* strain as well as DSM 34653 were subjected to a protein similarity search against a database containing 4,236 experimentally validated virulence factors (protein core dataset downloaded from the Virulence Factor Database (VFDB) on April 16, 2024) using the Basic Local Alignment Search Tool (BLAST). The blastp algorithm (v2.12.0+) with a word-size of 7 was used for the alignment search.

*Antimicrobial resistance analysis*

RefSeq genomes of the four *P. aeruginosa* strains included in the virulence analysis (see the *Virulence factor analysis* section) and the genome assembly of DSM 34653 were subjected to antimicrobial resistance analysis using the Resistance Gene Identifier tool (rgi v6.0.3) and the Comprehensive Antibiotic Resistance Database (CARD v3.2.9 containing 5,194 reference sequences, downloaded on April 17, 2024). rgi main with default parameters (Strict and Perfect hits were reported) was performed.

*16S rRNA extraction and mapping*

Six 16S ribosomal RNA (rRNA) genes were identified in the assembled genome of DSM 34653. The DNA sequences corresponding to the six 16S rRNA genes were extracted from the Prokka genome annotation output and compared to each other and to a database from NCBI containing curated 16S rRNA gene sequences from bacteria and archaea type strains using BLAST. The blastn algorithm (v2.14.1+) with a word-size of 11 was used for the nucleotide alignment search. Search was performed on April 3, 2024.

As the six 16S rRNA gene sequences are 100% identical to each other, the DNA sequence corresponding to one of these is encoded by **SEQ ID NO: 1** (1,532 nucleotides).

*Extraction and analysis of DSM 34653-specific DNA sequences*

DNA sequences with no or little overlap with the strain genomes of *P. wadenswilerensis* were extracted from the DSM 34653 genome assembly using samtools (v.1.10) and subsequently compared to either the complete nucleotide collection (nt database) from NCBI containing GenBank+EMBL+DDBJ+PDB+RefSeq sequences using BLAST. The blastn algorithm (v2.14.1+) with a word-size of 11 was used for all nucleotide alignment searches. Searches were performed on April 9-11, 2024.

Three long extracted DNA sequences 14,515-16,414 nucleotides) are annotated as **SEQ ID NOs: 2-4.**

Short DNA sequences (303-2,779 nucleotides) extracted from the genome assembly are annotated as **SEQ ID NOs: 5-17.**

*Results*

Initial taxonomic classification of Illumina and ONT sequencing reads obtained from the bacterial strain DSM 34653 revealed that 99.89% and 99.93% of all quality-controlled reads were assigned to the genus *Pseudomonas,* respectively. Analysis at species level revealed assignments to several characterized and uncharacterized *Pseudomonas* species **(Table 2)** where the majority of the sequencing data were assigned towards the latter (representing a total assignment of 44.84% and 41.37% of Illumina and ONT data, respectively). Amongst the characterized *Pseudomonas* species, top assignments were observed towards *P. wadenswilerensis, P. donghuensis* and *P. amygdali* **(Table 2).** Collectively, these data suggest that DSM 34653 belongs to the genus *Pseudomonas* and that it further shares taxonomic and genetic relatedness with several *Pseudomonas* species. Further insight is therefore needed to delineate DSM 34653.

*Table 2: Top taxonomic assignments of all classified Illumina or ONT reads at species level. The uncharacterized Pseudomonas spp. row indicates assignments towards several Pseudomonas species of uncharacterized status.*

| Species | Illumina assigned reads (%) | ONT assigned reads (%) |
|---|---|---|
| Uncharacterized *Pseudomonas* spp. | 44.84 | 41.37 |
| *Pseudomonas wadenswilerensis* | 23.67 | 23.27 |
| *Pseudomonas donghuensis* | 18.61 | 25.56 |
| *Pseudomonas amygdali* | 4.77 | 8.53 |

To obtain further biological insight into the taxonomic designation of DSM 34653, the genome of strain DSM 34653 was reconstructed *de novo.* A hybrid assembly approach was applied where long ONT reads were assembled into a non-fragmented de novo genome and the short Illumina reads were subsequently used for error-correction of the genome assembly at the nucleotide level. This approach allowed for generating a high-quality and complete *de novo* genome of strain DSM 34653. The complete genome is composed of one circular chromosome with characteristics depicted in **Table 3.**

*Table 3: Number of features associated with the genome assembly of DSM 34653. CDS=coding sequence; rRNA=ribosomal RNA; tRNA=transfer RNA; tmRNA=transfer-messenger RNA.*

| Genome feature | Size (bp) | CDS | rRNA | tRNA | tmRNA |
|---|---|---|---|---|---|
| Chromosome | 6,046,827 | 5,494 | 19 | 79 | 1 |

16S ribosomal RNA (rRNA) sequences have been extensively used to classify bacteria. Here, a threshold of 98.65% similarity at the 16S rRNA level has been recognized as the cutoff for delineating bacterial species. Six identical 16S rRNA genes were identified in DSM 34653. As these are 100% identical to each other, only the DNA sequence corresponding to one of the 16S rRNA genes identified in DSM 34653 is included here as **SEQ ID NO: 1.** Comparing **SEQ ID NO: 1** to the NCBI 16S ribosomal type strain database revealed a sequence identity of 99.80% along with 100% sequence coverage towards the 16S rRNA gene identified in the type strain of *Pseudomonas donghuensis* (HYS), indicating that DSM 34653 may be related to *P. donghuensis* which the taxonomic assignment initially also suggested.

Although the 16S rRNA gene region contains hypervariable regions that can be used for taxonomic discrimination, the analysis of 16S rRNA alone ignores the genome-wide variability. Thus, to further delineate the taxonomic identity of DSM 34653, average nucleotide identity (ANI) values were computed for DSM 34653 in a comparative analysis against all available *Pseudomonas* reference genomes located at NCBI (n=368 as of April 4, 2024, excluding atypical genomes). Average nucleotide identity (ANI) is a measure of the mean nucleotide identity of orthologous gene pairs that are shared between two microbial genomes and has been shown to be a robust measure when delineating bacterial genomes at species level. Large-scale studies have shown that microorganisms showing ≥95% ANI belong to the same species taxonomy. Comparing the assembled genome of DSM 34653 to all reference *Pseudomonas* genomes revealed a maximum ANI value of 93.07% towards the type strain of *Pseudomonas wadenswilerensis* (strain CCOS 864) **(Table 4, Figure 1).** Thus, the comparative analysis revealed no ANI values at or above the species threshold at 95% ANI, demonstrating that DSM 34653 should be considered as a novel bacterial species belonging to the genus *Pseudomonas*.

*Table 4: Average nucleotide identity (ANI) analysis of DSM 34653 genome assembly compared to all Pseudomonas reference genomes downloaded from NCBI (n=368, excluding atypical genomes, as of April 3, 2024). Scores ≥90% ANI are depicted here.*

| *Pseudomonas* reference | RefSeq assembly | ANI (%) |
|---|---|---|
| *P. wadenswilerensis* strain CCOS 864 | GCF_900497695.1 | 93.07 |
| *P. donghuensis* strain P482 | GCF_000696345.2 | 92.34 |
| *P. tructae* strain SNU WT1 | GCF_004214895.1 | 90.78 |
| *P. rubra* strain ID1025 | GCF_028656935.1 | 90.62 |

While ANI is a robust measure for delineating organisms at species, or even strain, level, the methodology is based on a pair-wise comparison of orthologous gene pairs that are shared between microbial genomes. To explore whether DNA sequence information located outside of orthologous gene regions might contribute with additional information on the specification of DSM 34653, whole genome phylogenies, representing measures of evolutionary distantness, were inferred on the basis of all available *Pseudomonas* reference genomes (n=368) and visualized as phylogenetic trees **(Figure 2).** Zooming into the phylogenetic clade containing DSM 34653 revealed that DSM 34653 is genetically closest to *P. wadenswilerensis, P. donghuensis, P. tructae* and *P. rubra* **(Figure 2,** tree to the right), which is in line with the ANI analysis **(Table 4).** Phylogenetic analysis of DSM 34653 and all available *P. wadenswilerensis, P. donghuensis, P. tructae* and *P. rubra* strain genomes (n=14) moreover revealed that DSM 34653 forms a separate phylogenetic branch, strongly indicating that DSM 34653 has diverged into a new bacterial lineage representative of a novel *Pseudomonas* species **(Figure 3).** To further define DSM 34653 at the genome level, genome-to-genome comparisons were performed between DSM 34653 and strain genomes of *P. wadenswilerensis* (n=2). Genomic regions in the genome assembly of DSM 34653 with no or low coverage in the strains of *P. wadenswilerensis* were extracted and subjected to a broad sequence similarity search. This identified several longer genomic regions matching ≤67% towards regions found in other prokaryotic genomes **(Table 5, SEQ ID NOs: 2-4).** Thus, these regions seem highly specific for DSM 34653.

*Table 5: Maximum percent sequence coverage and maximum percent sequence identity (relating to the max. seq. coverage percentage) from a nucleotide sequence similarity search of **SEQ ID NOs: 2-4** against the nucleotide database from the National Center for Biotechnology Information (NCBI). Search was performed on April 9, 2024. nt; nucleotides.*

| SEQ ID NO | Length (nt) | Max. seq. coverage (%) | Max. seq. identity (%) |
|---|---|---|---|
| #2 | 14,515 | 20 | 77.26 |
| #3 | 16,414 | 67 | 78.67 |
| #4 | 14,940 | 49 | 74.57 |

Importantly, several long open reading frames encoding hypothetical proteins were discovered within these long genomic regions, indicating that **SEQ ID NOs: 2-4** might be of functional importance to DSM 34653. Such regions are included for each longer region **(SEQ ID NOs: 2-4)** as **SEQ ID NOs: 5-13 (Table 6).**

*Table 6: Maximum percent sequence coverage and maximum percent sequence identity (relating to the max. seq. coverage percentage) from a nucleotide sequence similarity search of **SEQ ID NOs: 5-13** against the nucleotide database from the National Center for Biotechnology Information (NCBI). Search was performed on April 9-10, 2024. nt; nucleotides.*

| SEQ ID NO | Located in | Length (nt) | Max. seq. coverage (%) | Max. seq. identity (%) |
|---|---|---|---|---|
| #5 | | 1,713 | 15 | 67.52 |
| #6 | SEQ ID NO: 2 | 867 | 70 | 65.32 |
| #7 | | 1,374 | 4 | 81.82 |
| #8 | | 1,140 | 50 | 68.36 |
| #9 | SEQ ID NO: 3 | 408 | 16 | 83.58 |
| #10 | | 708 | 58 | 65.28 |
| #11 | | 2,779 | 75 | 67.64 |
| #12 | SEQ ID NO: 4 | 447 | 71 | 67.15 |
| #13 | | 303 | 68 | 70.67 |

Four additional, and randomly selected, DNA sequences, encoding hypothetical proteins and located outside of **SEQ ID NOs: 2-4** are further enclosed as **SEQ ID NOs: 14-17 (Table 7).** Comparing each of **SEQ ID NOs: 5-17** to the nucleotide database at NCBI using BLAST revealed that 4-85% of the extracted sequences could be aligned to other prokaryotic genomic regions with sequence identities of 65.28-95.45% **(Table 6 and 7).** Thus, **SEQ ID NOs: 5-17** seem highly selective for DSM 34653.

*Table 7: Maximum percent sequence coverage and maximum sequence identity (relating to the max. seq. coverage percentage) from a nucleotide sequence similarity search of **SEQ ID NOs: 14-17** against the nucleotide database from the National Center for Biotechnology Information (NCBI). Search was performed on April 11, 2024. nt; nucleotides.*

| SEQ ID NO | Length (nt) | Max. seq. coverage (%) | Max. seq. identity (%) |
|---|---|---|---|
| #14 | 1,077 | 36 | 66.33 |
| #15 | 714 | 43 | 95.45 |
| #16 | 828 | 83 | 70.08 |
| #17 | 780 | 85 | 75.85 |

To assess the potential properties of DSM 34653 that may be beneficial for agriculture, the genome of DSM 34653 was further explored. Interestingly, the assembled genome of DSM 34653 contains genes relating to the solubilization and mineralization of both inorganic *(gcd, ppx* and *ppa)* and organic *(aphA)* phosphate sources, phosphate transportation *(pitA, pstB, pstS)* and phosphate-deprivation responses *(phoB, phoR, phoU),* indicating a strong genomic potential of DSM 34653 to increase the pool of available phosphate, even under phosphate-scarce conditions, that plants can utilize for growth. Phosphate-solubilizing capabilities of DSM 34653 are supported by *in vitro* assays presented in **Example 4.** Interestingly, DSM 34653 contains the *hcnABC* gene cluster encoding the enzyme involved in the synthesis of hydrogen cyanide (HCN); a secondary metabolite that has been linked to biocontrol properties by suppressing plant pathogens. The ability of DSM 34653 to suppress the growth of phytopathogenic fungi *in vitro* is presented in **Example 3.**
Several pseudomonads are known to cause a variety of opportunistic infections and diseases in humans, including *P. aeruginosa, P. fluorescens* and *P. putida. P. aeruginosa* strains account for most infections, and many of these are also resistant to commonly used antibiotics. *P. aeruginosa* produces several virulence factors, including toxins that via secretion and injection systems can enter host cells and cause cellular death. Comparing translated nucleotide sequences of DSM 34653 and four virulent *P. aeruginosa* strains (PAO1, UCBPP PA14, LESB58 and PA7) to a protein database containing experimentally validated virulence factors (core dataset containing 4,236 virulence factors, downloaded from the Virulence Factor Database (VFDB) on April 16, 2024) revealed that gene products of DSM 34653 showed a significant (≥90% sequence overlap and ≥90% sequence identity) overlap with seven putative virulence factors **(Table 8)** while gene products of the included *P. aeruginosa* strains matched towards 215-326 virulence factors **(Figure 4).** Importantly, the maximum sequence identity between a gene encoding a virulence factor and genes annotated in DSM 34653 was 94.36%, whereas 100% sequence identities were reported for virulence hits in all four P. *aeruginosa* strains (10-98.50% of all hits were associated with 100% sequence identities) **(Figure 4)**, suggesting that virulence hits in *P. aeruginosa* strains are more valid. The virulence factors discovered in DSM 34653 relate to flagellar motility, scavenging of iron (via pyoverdine secretion), biofilm formation and secretory systems **(Table 8).** While such properties are relevant for pathogenic microorganisms, the total number of virulence factors found in DSM 34653 is low, at least compared to hypervirulent microorganisms, such as *P. aeruginosa* strains. Moreover, and importantly, the gene encoding exotoxin A, which has been reported as the most toxic virulence factor and is secreted by many clinically relevant *P. aeruginosa* strains, is not present in the genome assembly of DSM 34653, suggesting a non-pathogenic nature of DSM 34653. In addition, the virulence factors listed in **Table 8** can also be viewed as important factors for improving plant growth by for example facilitating iron uptake in plants and secretion of plant-beneficial biomolecules such as HCN, demonstrating that the function of individual virulence factors are multifaceted. Plant growth-promoting capabilities of DSM 34653 are presented in **Example 5.** Finally, genome mining further revealed no presence of the rhizoxin-biosynthetic gene cluster, including both *rhi* and *rzx* gene loci, which encode metabolites that may be toxic to human health.

***Table 8:*** *Virulence factor analysis of DSM 34653 by comparing translated nucleotide sequences to the protein core dataset from the Virulence Factor Database (VFDB, downloaded on April 16, 2024). The listed virulence factors represent significant hits in DSM 34653. VF=virulence factor.*

| VF category | VF | VF description |
|---|---|---|
| Motility | Flagella | Flagellar motor switch protein FliM |
| Motility | Flagella | Flagellar synthesis regulator FleN |
| Motility | Flagella | Chemotaxis protein CheY |
| Nutritional/ Metabolic factor | Pyoverdine | Extracytoplasmic-function sigma-70 factor |
| Biofilm | Alginate | Alginate biosynthesis protein AlgZ/FimS |
| Effector delivery system | HSI-1 | Type VI secretion system tubule-forming protein VipA |
| Effector delivery system | HSI-1 | Type VI secretion system tubule-forming protein VipB |

Drug-resistant infections caused by antimicrobial resistance are a global burden and one of the leading threats to public health. Antimicrobial resistance occurs when microorganisms no longer respond to antibiotic drugs through the acquirement of antibiotic resistance traits. Performing an antimicrobial resistance analysis of DSM 34653 identified seven putative antimicrobial resistance genes of which none importantly were perfect hits, i.e., having a complete sequence overlap along with 100% identity towards a resistance gene **(Table 9).** Comparing this to the pattern of antimicrobial resistance genes present in the four virulent *P. aeruginosa* strains (PAO1, UCBPP PA14, LESB58 and PA7), that were also included in the virulence analysis, 55-62 resistance genes were detected in each strain. A heatmap was obtained representing sequence similarities (% identity) of antimicrobial resistance (AMR) genes and the genome assembly of DSM 34653 and four pathogenic *Pseudomonas aeruginosa* strains (PAO1, UCBPP PA14, LESB58 or PA7). 78 significant and unique AMR genes were found across all genomes analyzed **(Data not shown).**

Of these, *rsmA* and *soxR* (the top2 hits in DSM 34653 **(Table 9))** were detected as perfect hits in three-four *P. aeruginosa* strains. Moreover, 92-95% of all resistance genes identified in the included *P. aeruginosa* strains had sequence identities above the best hit identity (80.65%) in DSM 34653. In addition, studies have shown the presence of *rsmA,* encoding a global posttranscriptional regulator, in non-pathogenic bacteria. While prediction of a phenotype solely on the basis of genome information is still in its infancy, the virulence factor and antimicrobial resistance analyses collectively suggest that DSM 34653 can be regarded as a commensal bacterium.

*Table 9: Antimicrobial resistance analysis of DSM 34653 by comparing translated nucleotide sequences to The Comprehensive Antibiotic Resistance Database (CARD, downloaded on April 17, 2024). The listed hits represent the corresponding antimicrobial resistance genes (ARGs), percent overlap (coverage) between translated nucleotide sequences and protein sequences from CARD along with percent identity and the associated antibiotic drug class.*

| Best hit ARG | Best hit coverage (%) | Best hit identity (%) | Drug class |
|---|---|---|---|
| *rsmA* | 101.64 | 80.65 | fluoroquinolone antibiotic; diaminopyrimidine antibiotic; phenicol antibiotic |
| *soxR* | 98.72 | 70.55 | fluoroquinolone antibiotic; cephalosporin; glycylcycline; penam; tetracycline antibiotic; rifamycin antibiotic; phenicol antibiotic; disinfecting agents and antiseptics |
| *adeF* | 100.85 | 66.79 | fluoroquinolone antibiotic; tetracycline antibiotic |
| *adeF* | 98.39 | 43.4 | fluoroquinolone antibiotic; tetracycline antibiotic |
| *adeF* | 100.00 | 66.89 | fluoroquinolone antibiotic; tetracycline antibiotic |
| *vanH* gene in *vanB* cluster | 101.86 | 37.27 | glycopeptide antibiotic |
| *adeF* | 99.15 | 41.22 | fluoroquinolone antibiotic; tetracycline antibiotic |

## *Conclusion*

Phylogenetic analyses and genome comparisons revealed that DSM 34653 can be considered as a novel bacterial species belonging to the genus *Pseudomonas.* Interestingly, DSM 34653 encodes several properties that are beneficial to agricultural farming, including phosphate solubilizing capabilities. In addition, DSM 34653 contains a complete biosynthetic gene cluster matching towards the *hcnABC* gene cluster that encodes the secondary metabolite hydrogen cyanide which has been suggested to act as a broad-spectrum biocontrol agent. As DSM 34653 is classified as a novel bacterial species, virulence and antimicrobial resistance properties were assessed. These analyses revealed the presence of few virulence and antimicrobial resistance genes; however, these were associated with lower sequence identities compared to gene hits identified in pathogenic P. *aeruginosa* strains.

Several DNA sequences containing open reading frames and that have high specificity for DSM 34653 were identified; three long genomic regions (14,515-16,414 nucleotides, **SEQ ID NOs: 2-4**) wherein short DNA sequences (303-2,779 nucleotides) were extracted **(SEQ ID NOs: 5-13)** as well as four short DNA sequences (714-1,077 nucleotides) identified outside of the three long genomic regions **(SEQ ID NOs: 14-17).** Combined or alone, these DNA sequences may be used to identify DSM 34653 at the DNA sequence level.

## Example 3 - Assessing biocontrol properties of DSM 34653

## *Aim of study*

To demonstrate the ability of DSM 34653 to control growth of common fungal plant pathogens.

## *Materials and methods*

Dual culture assays were used to determine the antifungal activity of DSM 34653. DSM 34653 was co-cultured on PDApotato dextrose agar (PDA), grass agar (GA), or oatmeal agar (OA) with three isolates of fungal pathogens; *Gaeumannomyces graminis var. tritici* (isolate CBS 450.77), *Pyrenophora teres f. teres* (isolate CP2189), *Zymoseptoria tritici* (isolate IPO323), *Fusarium oxysporum* (isolate CBS 619.87). These four fungi are causing some of the major diseases in agricultural crops; wheat root rot (take-all), barley net blotch, septoria tritici blotch, and vascular wilts in a variety of crops. A spot on lawn dual culture assay was used to determine the antifungal activity. Petri dishes containing OA, GA, or PDA were prepared with a lawn of fungi using a cotton swab covered in fungal spores. The spores were distributed by striking the swab methodically across the agar surface in one direction ensuring close and even distribution of spores. Subsequently the swab was struck perpendicularly to the initial direction further enhancing the uniformity of the lawn. A solution with a concentration of 3 McF of DSM 34653 was prepared in 1:10 PBS. 10 $\mu$L of the solution was spotted onto each petri dish already prepared with fungal spores in replicates of three. As benchmarking control the *Bacillus amyloliquefaciens* QST 713 strain was included (Bio1). This strain is the active ingredient in a commercial microbial biofungicide. The active microorganisms in this product were isolated and included in the test on equal footing with DSM 34653. Inhibition was qualitatively evaluated by the presence of a halo zone with no fungal growth on the 'lawn'.

The assay was performed at 20°C, to ensure optimal conditions for fungal growth. Inhibition was evaluated on day 8, except DSM 34653 on *Zymoseptoria tritici,* which was evaluated on day 10.

*Results*

DSM 34653 inhibited the growth of all four phytopathogenic fungi *(Gaeumannomyces graminis var. tritici* (isolate CBS 450.77), *Pyrenophora teres f.* teres (isolate CP2189), *Zymoseptoria tritici* (isolate IPO323), *Fusarium oxysporum* (isolate CBS 619.87)) when grown in a dual culture assay with a spot on lawn design **(Figure 5** and **Table 10).** Similar results were observed with the benchmarking control (Bio1), a biofungicide product already on the market, except it did not inhibit growth of *Pyrenophora teres f. teres.* These findings underscore the potential of DSM 34653 as a biofungicide for mitigating fungal attacks on agricultural crops.

*Table 10. Assessment of fungal growth inhibition by bacterial products. Inhibition was assessed qualitatively; '÷' indicates no inhibition of fungal growth, while '+' indicates inhibition of fungal growth around the bacterial colony. Fungi were cultivated on oatmeal agar (OA), grass agar (GA), or potato dextrose agar (PDA).*

| Phytopathogenic fungi | Disease | Crop | Nutrient agar | Inhibition | | |
|---|---|---|---|---|---|---|
| | | | | Neg. control | DSM 34653 | Bio1 |
| *Gaeumannomyces graminis* | Take-all | Wheat | OA | ÷ | + | + |
| *Pyrenophora teres* | Net blotch | Barley | GA | ÷ | + | ÷ |
| *Zymoseptoria tritici* | Septoria tritici blotch | Wheat | PDA | ÷ | + | + |
| *Fusarium oxysporum* | Root rot | | OA | ÷ | + | + |

*Conclusion*

Addressing fungal crop diseases is crucial for maintaining global food security and sustainable agricultural practices. DSM 34653 was shown to have antifungal activity against four common phytopathogenic fungi *Gaeumannomyces graminis* var. *tritici* (isolate CBS 450.77), *Pyrenophora teres f. teres* (isolate CP2189), *Zymoseptoria tritici* (isolate IPO323), *Fusarium oxysporum* (isolate CBS 619.87) when evaluated in a dual culture assay. Based on these results DSM 34653 could hold potential for mitigating crop losses caused by fungal diseases, thereby enhancing agricultural productivity and sustainability.

Without being bound by theory, DSM 34653 may be used in the treatment of fungal infections in humans, and thus suitable for use as a medicament.

**Example 4** - **DSM 34653 has mineral-dissolving properties at low, medium and high temperatures**

*Aim of study*

The aim is to demonstrate the efficacy of DSM 34653 as a biostimulant in increasing the availability of common plant nutrients under low, medium, and high temperature conditions.

*Materials and methods*

To demonstrate the ability of DSM 34653 to increase the availability of nitrogen, phosphorous, and potassium it was cultivated on different solid media: nitrogen-free agar; Jensen medium, insoluble phosphate agar; Pikovskaya agar, and insoluble potassium agar; Aleksandrow agar). These three solid media are recognized as specific for detection and cultivation of nitrogen-fixing, phosphate-solubilizing, and potassium solubilizing soil microorganisms. Growth on Jensen agar indicates nitrogen-fixating abilities, as this medium is free of nitrogen. A halo zone on Pikovskaya, and Aleksandrow agar indicates solubilization of calcium phosphate ($Ca_3(PO_4)_2$ and potassium alumino-silicate respectively. The solubilization capacity was evaluated by calculating the solubilization index (SI, $Area_{Halo}/Area_{Colony}$). To demonstrate their activity dependent on temperature the experiment was performed at low (5°C), medium (15°C), and high (25°C) temperatures. Typically, spring seeding will occur at soil temperatures around 8°C in Northern Europe, and activity at lower temperatures is therefore of great importance at this altitude.

As benchmarking control the *Bacillus amyloliquefaciens* QST 713 strain was included (Bio1). This strain is the active ingredient in a commercial microbial biofungicide

The active strain of two well known biostimulants were included in the experiment as benchmarking controls on equal footing with DSM 34653. These strains were *Bacillus amyloliquefaciens* QST 713 (Bio1) and *Bacillus atrophaeus* Abi05 (Bio2). A fluid culture of each bacterial strain was prepared in a concentration of 0.5 McFarland. The solution was then spotted onto plates containing the different four different media in repetitions of three. The plates were incubated at either 5°C, 15°C, or 25°C. Colony and halo zone areas were measured each day using imageJ.

*Results*

The ability of DSM 34653 to increase the availability of nitrogen, phosphate or potassium was evaluated using a solid media strategy where each bacterial colony and/or halo zone area was quantified and furthermore compared to bacterial strains isolated from commercial biostimulant and biofungicide products **(Figure 6)**. DSM 34653 was actively growing on each respective media across the entire temperature span (low (5°C), medium (15°C, and high (25°C)) and was the only strain that could grow and/or exert activity at 5°C. Moreover, DSM 34653 exerted mineral-dissolving properties at all temperatures **(Figure 6B-C)** while the bacterial control strains isolated from commercial products only demonstrated little **(Figure 6B)** or no **(Figure 6C)** capacity towards dissolving phosphate or potassium, respectively. The solubilization index (SI) of DSM 34653 on day 7 on Pikovskaya agar was in the range 2 and 9, wherein the SI was about 2.3 at 5°C, about 8.6 at 15°C, and about 4.7 at 25°C **(Figure 6B)**. The SI of DSM 34653 on day 10 on Aleksandrow agar was in the range 2 and 5, wherein the SI was about 2.2 at 5°C, about 2.2 at 15°C, and about 4.8 at 25°C **(Figure 6)**.

This suggests that DSM 34653 has a better capacity towards dissolving minerals which are otherwise unavailable for plants.

*Conclusion*

Soil phosphate and potassium exist to a large extent in insoluble complexes bound to insoluble inorganic minerals, which are unavailable for plants. Thus, solubilization of these minerals may improve mineral availability and thereby has the potential to promote plant growth. Only DSM 34653 was able to grow and display bacterial activity at 5°C, demonstrating that DSM 34653 has a high potential for supplying crops in the field with otherwise unavailable nutrients through the entire plant life cycle from as early as germination in cold soil until maturation and harvest.

**Example 5** - **Seed coat containing DSM 34653 promotes early plant growth**

*Aim of study*

The scope of this example is to demonstrate the stimulatory properties of DSM 34653, when applied as a seed coat, on early plant growth *in vitro* in spring barley; a typical agricultural crop.

*Materials and methods*

Spring barley seeds (*Hordenum vulgare* 'Skyway') were manually coated with a bacterial solution containing $1.47 \times 10^9$ CFU DSM 34653 per mL (n=70 seeds). The following was used for coating 5 g of seeds: 12.5 $\mu$L DSM 34653 ($1.47 \times 10^9$ CFU/mL) + 62.5 $\mu$L of demineralized water. Untreated seeds were used as a control (n=70 seeds). In a third experimental group, seeds were treated with both DSM 34653 and a commercial chemical fungicide containing the following active substances: tebuconazol (20 g/L) and prothioconazol (150 g/L) (n=70 seeds).

The coating process was performed in a 50 mL conical tube where a bacterial solution containing DSM 34653 (diluted in water) or a commercial chemical fungicide were added to the seeds. Tubes were thoroughly shaken to ensure even distribution of bacteria or fungicide on seeds. Seeds were afterwards sown into trays and stored indoors with a day-night cycle of 16h/8h at $20 \pm 2$°C. The emergence percentage of seedlings was quantified five days following sowing as (# of emerged seedlings / # seeds sown) x 100%.

*Results*

Treating spring barley seeds with a seed coat containing a bacterial solution of DSM 34653 before sowing increased the emergence at five days following sowing with 19% compared to non-treated seeds **(Figure 7).** Compared to seeds that were treated with both a commercial chemical fungicide and DSM 34653, DSM 34653 coating alone increased the emergence with 41%. Interestingly, the emergence of seedlings was higher among non-treated seeds compared to seeds that were treated with both commercial chemical fungicide and DSM 34653 (16%), suggesting that fungicide treatment prior to sowing is associated with lower emergence percentages.

*Conclusion*

Spring barley is among the most common grown cereal crops in Denmark. Treating spring barley seeds with a bacterial solution containing DSM 34653 before sowing demonstrates that DSM 34653 can enhance the emergence of seedlings, also when seeds have been treated with a commercially available chemical fungicide, suggesting that an increased yield also may be obtained.

**Sequence listing**

| SEQ ID NO: 1 | Strain DSM 34653 16S ribosomal DNA sequence. |
|---|---|
| SEQ ID NOs: 2-4 | Strain DSM 34653 long DNA sequences |
| SEQ ID NOs: 5-17 | Strain DSM 34653 short DNA sequences<br>SEQ ID NOs: 5-13 are located within the long genomic regions (SEQ ID NOs: 2-4)<br>SEQ ID NOs: 14-17 are located outside the long genomic regions (SEQ ID NOs: 2-4) |

**Claims**

1.  A bacteria or biologically pure bacterial culture

    - being *Pseudomonas,* DSM 34653 deposited with the DSMZ [LEIBNIZ-INSTITUT DSMZ-DEUTSCHE SAMMLUNG VON MIKROORGANISMEN UND ZELLKULTUREN GmbH, Inhoffenstr. 7 B, D-38124, Braunschweig, Germany] on 24 May 2023.

2.  The bacteria or biologically pure bacterial culture according to claim 1, being

    - capable of solubilizing calcium phosphate at 5°C, and/or iron phosphate at 5°C and/or potassium aluminum silicate at 5°C; and/or
    - capable of inhibiting one or more phytopathogenic fungi.

3.  The bacteria or biologically pure bacterial culture according to claim 2, wherein the one or more phytopathogenic fungi are selected from the group consisting of *Gaeumannomyces graminis* var. *tritici, Pyrenophora teres f. teres, Zymoseptoria tritici,* and *Fusarium oxysporum.*

4.  The bacteria or biologically pure bacterial culture according to claim 2, wherein the solubilization of calcium phosphate, iron phosphate, and/or potassium aluminum silicate at 5°C is at a level of at least 40% to, equal to and/or above a level of solubilization at 15°C and/or 25°C.

5.  A fertilizer and/or inoculant and/or biostimulant and/or biofungicide and/or antimicrobial composition comprising the bacteria or biologically pure bacterial culture according to any of claims 1-4.

6.  The fertilizer and/or inoculant and/or biostimulant composition according to claim 5, further comprising other microorganisms, such as other microorganisms able to function as a biostimulant.

7.  A coating composition, preferably a seed coating composition, comprising the bacteria or biologically pure bacterial culture according to any of the claims 1-4 and/or the composition according to any of claims 5-6.

8.  The coating composition according to claim 7, the coating composition comprises a biopolymer promoting adherence to a plant seed.

9.  A plant seed coated with the composition according to any of claims 5-6 or coated with a coating composition according to any of claims 7-8.

10. Use of a bacteria or biologically pure bacterial culture according to any of claims 1-4, the composition according to any of claims 5-6 or the coating composition according to any of claims 7-8, as a plant growth promoting agent, such as a fertilizer or inoculum or biostimulant, preferably at a temperature below 15°C, such as below 10°C, preferably between 2°C and 8°C, more preferably around 5°C.

11. The use according to claim 10, wherein the use as a plant growth-promoting agent is for:

- solubilizing inorganic minerals or salts, such as phosphate, and/or potassium, and/or iron, and/or aluminium, and/or zinc, and/or manganese; and/or
- chelating inorganic minerals such as iron, zinc, manganese, copper; and/or
- allowing for growth under low soil nitrogen conditions; and/or
- fixating atmospheric nitrogen; and/or
- increasing plant growth, such as plant length, leaf diameter and/or root length; and/or
- improving germination of the seeds; and/or
- improving emergence of the plants; and/or
- increasing biomass; and/or
- increasing growth; and/or
- increasing crop yield; and/or
- inducing anti-fungal effects; and/or
- increasing harvest yield; and/or
- increasing hectoliter weight; and/or
- increasing protein content; and/or
- increasing protein yield; and/or
- increasing oil content; and/or
- increasing oil yield; and/or
- reducing mycotoxin contamination; and/or
- reducing seed-borne fungal or bacterial contamination; and/or
- improving taste, smell or appearance; and/or
- replacing or substituting any chemical product used in plant production while retaining one or more desirable plant parameters.

12. The use according to any of claims 10 or 11, wherein the use takes place at field temperatures in the range -10°C to 15°C, such as -5°C to 15°C, preferably 0 to 15°C, more preferably such as 2 to 10°C, such 2 to 8°C.

13. A method for stimulating plant growth comprising applying the bacteria or biologically pure bacterial culture according to any of claims 1-4, the composition according to any of claims 5-6 or the coating composition according to any of claims 7-8 to a plant, plant seed, a sowing forrow, soil and/or plant growth medium, preferably at a temperature below 15°C, such as below 10°C, preferably between 2°C and 8°C, more preferably around 5°C.

14. A kit of parts for stimulating plant growth comprising

- a first container comprising the bacteria or biologically pure bacterial culture according to any of claims 1-4, the composition according to any of claims 5-6 and/or the coating composition according to any of claims 7-8; and
- instructions for applying the bacteria or biologically pure bacterial culture according to any of claims 1-4, the composition according to any of claims 5-6 and/or the coating composition according to any of claims 7-8 to plants, plant seeds, or a plant growth medium, preferably at a temperature below 15°C, such as below 10°C, preferably between 2°C and 8°C, more preferably around 5°C.

15. A bacteria or biologically pure bacterial culture according to any of claims 1-4, a composition according to any of claims 5-6 or a coating composition according to any of claims 7-8 for use as a medicament.

**Patentansprüche**

1. . Bakterium oder biologisch reine Bakterienkultur

- der Gattung Pseudomonas, DSM 34653, hinterlegt bei der DSMZ [LEIBNIZ-INSTITUT DSMZ-DEUTSCHE SAMMLUNG VON MIKROORGANISMEN UND ZELLKULTUREN GmbH, Inhoffenstr. 7 B, D-38124, Braunschweig, Deutschland] am 24. Mai 2023.

**2.** . Bakterium oder biologisch reine Bakterienkultur nach Anspruch 1, wobei diese

- zum Lösen von Kalziumphosphat bei 5 °C und/oder Eisenphosphat bei 5 °C und/oder Kaliumaluminiumsilikat bei 5 °C ausgelegt ist; und/oder
- zum Hemmen von einem oder mehreren phytopathogenen Pilzen ausgelegt ist.

**3.** . Bakterium oder biologisch reine Bakterienkultur nach Anspruch 2, wobei die einen oder mehrere phytopathogenen Pilze aus der Gruppe ausgewählt werden, bestehend aus *Gaeumannomyces graminis* var. *tritici, Pyrenophora teres* f. *teres, Zymoseptoria tritici,* und *Fusarium oxysporum.*

**4.** . Bakterium oder biologisch reine Bakterienkultur nach Anspruch 2, wobei die Löslichkeit von Kalziumphosphat, Eisenphosphat und/oder Kaliumaluminiumsilikat bei 5 °C auf einem Niveau von mindestens 40 % bis zu, gleich und/oder über einem Löslichkeitsniveau bei 15 °C und/oder 25 °C liegt.

**5.** . Düngemittel- und/oder Impfmittel- und/oder Biostimulans- und/oder Biofungizid- und/oder antimikrobielle Zusammensetzung, die das Bakterium oder die biologisch reine Bakterienkultur nach einem der Ansprüche 1 bis 4 umfasst.

**6.** . Düngemittel- und/oder Impfmittel- und/oder Biostimulanszusammensetzung nach Anspruch 5, weiter umfassend andere Mikroorganismen, wie andere Mikroorganismen, die als Biostimulans fungieren können.

**7.** . Beschichtungszusammensetzung, bevorzugt eine Samenbeschichtungszusammensetzung, die das Bakterium oder die biologisch reine Bakterienkultur nach einem der Ansprüche 1 bis 4 und/oder die Zusammensetzung nach einem der Ansprüche 5 bis 6 umfasst.

**8.** . Beschichtungszusammensetzung nach Anspruch 7, wobei die Beschichtungszusammensetzung ein Biopolymer umfasst, das die Haftung an einem Pflanzensamen fördert.

**9.** . Pflanzensamen, beschichtet mit der Zusammensetzung nach einem der Ansprüche 5 bis 6 oder beschichtet mit einer Beschichtungszusammensetzung nach einem der Ansprüche 7 bis 8.

**10.** . Verwendung eines Bakteriums oder einer biologisch reinen Bakterienkultur nach einem der Ansprüche 1 bis 4, der Zusammensetzung nach einem der Ansprüche 5 bis 6 oder der Beschichtungszusammensetzung nach einem der Ansprüche 7 bis 8, als pflanzenwachstumsförderndes Mittel, wie ein Düngemittel oder ein Impfmittel oder ein Biostimulans, bevorzugt bei einer Temperatur unter 15 °C, wie unter 10 °C, bevorzugt zwischen 2 °C und 8 °C, bevorzugter um 5 °C.

**11.** . Verwendung nach Anspruch 10, wobei die Verwendung als pflanzenwachstumsförderndes Mittel zu Folgendem dient:

- Lösen anorganischer Mineralien oder Salze, wie Phosphat, und/oder Kalium, und/oder Eisen, und/oder Aluminium, und/oder Zink, und/oder Mangan; und/oder
- Chelatbilden anorganischer Mineralien wie Eisen, Zink, Mangan, Kupfer; und/oder
- Ermöglichen des Wachstums unter Bedingungen mit niedrigem Stickstoffgehalt im Boden; und/oder
- Fixieren von atmosphärischem Stickstoff und/oder
- Erhöhen des Pflanzenwachstums, wie beispielsweise der Pflanzenlänge, des Blattdurchmessers und/oder der Wurzellänge; und/oder
- Verbessern der Keimung der Samen und/oder
- Verbessern der Entstehung der Pflanzen und/oder
- Erhöhen der Biomasse und/oder
- Erhöhen des Wachstums; und/oder
- Erhöhen des Ernteertrags; und/oder
- Induzieren antimykotischer Wirkungen; und/oder
- Erhöhen des Ernteertrags; und/oder
- Erhöhen des Hektolitergewichts; und/oder
- Erhöhen des Proteingehalts; und/oder
- Erhöhen des Proteinertrags; und/oder

- Erhöhen des Ölgehalts; und/oder
- Erhöhen des Ölertrags; und/oder
- Verringern der Mykotoxinkontamination; und/oder
- Verringern der durch Saatgut übertragenen Pilz- oder Bakterienkontamination; und/oder
- Verbessern des Geschmacks, Geruchs oder Aussehens; und/oder
- Ersetzen oder Substituieren eines chemischen Produkts, das in der Anlagenproduktion verwendet wird, unter Beibehaltung eines oder mehrerer wünschenswerter Anlagenparameter.

12. . Verwendung nach einem der Ansprüche 10 oder 11, wobei die Verwendung bei Feldtemperaturen im Bereich von -10 °C bis 15 °C, wie -5 °C bis 15 °C, bevorzugt 0 bis 15 °C, bevorzugter wie 2 bis 10 °C, wie 2 bis 8 °C, erfolgt.

13. . Verfahren zum Anregen des Pflanzenwachstums, umfassend das Aufbringen des Bakteriums oder der biologisch reinen Bakterienkultur nach einem der Ansprüche 1 bis 4, die Zusammensetzung nach einem der Ansprüche 5 bis 6 oder die Beschichtungszusammensetzung nach einem der Ansprüche 7 bis 8 auf eine Pflanze, Pflanzensaatgut, eine Saatfurche, einen Boden und/oder ein Pflanzenwachstumsmedium, bevorzugt bei einer Temperatur unter 15 °C, wie unter 10 °C, bevorzugt zwischen 2 °C und 8 °C, bevorzugter um 5 °C.

14. . Satz von Teilen zum Anregen des Pflanzenwachstums, umfassend

- einen ersten Behälter, der das Bakterium oder die biologisch reine Bakterienkultur nach einem der Ansprüche 1 bis 4 umfasst, die Zusammensetzung nach einem der Ansprüche 5 bis 6 und/oder die Beschichtungszusammensetzung nach einem der Ansprüche 7 bis 8; und
- Anweisungen zum Aufbringen des Bakteriums oder der biologisch reinen Bakterienkultur nach einem der Ansprüche 1 bis 4, die Zusammensetzung nach einem der Ansprüche 5 bis 6 und/oder die Beschichtungszusammensetzung nach einem der Ansprüche 7 bis 8 auf Pflanzen, Pflanzensaatgut oder ein Pflanzenwachstumsmedium, bevorzugt bei einer Temperatur unter 15 °C, wie unter 10 °C, bevorzugt zwischen 2 °C und 8 °C, bevorzugter etwa 5 °C.

15. . Bakterium oder biologisch reine Bakterienkultur nach einem der Ansprüche 1 bis 4, eine Zusammensetzung nach einem der Ansprüche 5 bis 6 oder eine Beschichtungszusammensetzung nach einem der Ansprüche 7 bis 8 zur Verwendung als Arzneimittel.

## Revendications

1. Bactérie ou culture bactérienne biologiquement pure

- qui est Pseudomonas, DSM 34653 déposée auprès de la DSMZ [LEIBNIZ-INSTITUT DSMZ-DEUTSCHE SAMMLUNG VON MIKROORGANISMEN UND ZELLKULTUREN GmbH, Inhoffenstr. 7 B, D-38124, Braunschweig, Allemagne] le 24 mai 2023.

2. Bactérie ou culture bactérienne biologiquement pure selon la revendication 1, qui est

- apte à solubiliser le phosphate de calcium à 5 °C, et/ou le phosphate de fer à 5 °C et/ou le silicate d'aluminium et de potassium à 5 °C ; et/ou
- apte à inhiber un ou plusieurs champignons phytopathogènes.

3. Bactérie ou culture bactérienne biologiquement pure selon la revendication 2, dans laquelle les un ou plusieurs champignons phytopathogènes sont sélectionnés dans le groupe consistant en *Gaeumannomyces graminis* var. *tritici, Pyrenophora teres* f. *teres, Zymoseptoria tritici,* et *Fusarium oxysporum.*

4. Bactérie ou culture bactérienne biologiquement pure selon la revendication 2, dans laquelle la solubilisation du phosphate de calcium, du phosphate de fer et/ou du silicate d'aluminium et de potassium à 5 °C se trouve à un niveau d'au moins 40 % de, égal à et/ou supérieur à un niveau de solubilisation à 15 °C et/ou 25 °C.

5. Composition d'engrais et/ou d'inoculant et/ou de biostimulant et/ou de biofongicide et/ou d'antimicrobiens comprenant la bactérie ou la culture bactérienne biologiquement pure selon l'une quelconque des revendications 1 à 4.

6. Composition d'engrais et/ou d'inoculant et/ou de biostimulant selon la revendication 5, comprenant en outre d'autres micro-organismes, tels que d'autres micro-organismes capables de fonctionner comme biostimulant.

7. Composition d'enrobage, de préférence composition d'enrobage de semence, comprenant la bactérie ou la culture bactérienne biologiquement pure selon l'une quelconque des revendications 1 à 4 et/ou la composition selon l'une quelconque des revendications 5 à 6.

8. Composition d'enrobage selon la revendication 7, la composition d'enrobage comprend un biopolymère favorisant l'adhérence à une semence végétale.

9. Semence végétale enrobée de la composition selon l'une quelconque des revendications 5 à 6 ou revêtue d'une composition d'enrobage selon l'une quelconque des revendications 7 à 8.

10. Utilisation d'une bactérie ou d'une culture bactérienne biologiquement pure selon l'une quelconque des revendications 1 à 4, de la composition selon l'une quelconque des revendications 5 à 6 ou de la composition d'enrobage selon l'une quelconque des revendications 7 à 8, comme agent favorisant la croissance végétale, par exemple un engrais ou un inoculum ou un biostimulant, de préférence à une température inférieure à 15 °C, par exemple inférieure à 10 °C, de préférence entre 2 °C et 8 °C, plus préférablement autour de 5 °C.

11. Utilisation selon la revendication 10, dans laquelle l'utilisation comme agent favorisant la croissance végétale est pour :

- solubiliser les minéraux ou sels inorganiques, tels que le phosphate, et/ou
le potassium, et/ou le fer, et/ou l'aluminium, et/ou le zinc, et/ou le manganèse ; et/ou
- chélater les minéraux inorganiques, tels que le fer, le zinc, le manganèse, le cuivre ; et/ou
- permettre une croissance dans des conditions de faible teneur en azote dans le sol ; et/ou
- fixer l'azote atmosphérique ; et/ou
- augmenter la croissance végétale, par exemple la longueur des plantes, le diamètre des feuilles et/ou la longueur des racines ; et/ou
- améliorer la germination des semences ; et/ou
- améliorer l'émergence des plantes ; et/ou
- augmenter la biomasse ; et/ou
- augmenter la croissance ; et/ou
- augmenter le rendement des cultures ; et/ou
- induire des effets antifongiques ; et/ou
- augmenter le rendement des récoltes ; et/ou
- augmenter le poids en hectolitres ; et/ou
- augmenter la teneur en protéines ; et/ou
- augmenter le rendement en protéines ; et/ou
- augmenter la teneur en huile ; et/ou
- augmenter le rendement en huile ; et/ou
- réduire la contamination par les mycotoxines ; et/ou
- réduire la contamination fongique ou bactérienne d'origine semencière ; et/ou
- améliorer le goût, l'odeur ou l'apparence ; et/ou
- remplacer ou substituer tout produit chimique utilisé dans la production végétale tout en conservant un ou plusieurs paramètres végétaux souhaitables.

12. Utilisation selon l'une quelconque des revendications 10 ou 11, dans laquelle l'utilisation a lieu à des températures de champ dans la plage de -10 °C à 15 °C, par exemple -5 °C à 15 °C, de préférence 0 à 15 °C, plus préférablement par exemple 2 à 10 °C, par exemple 2 à 8 °C.

13. Procédé de stimulation de la croissance végétale comprenant l'application de la bactérie ou de la culture bactérienne biologiquement pure selon l'une quelconque des revendications 1 à 4, de la composition selon l'une quelconque des revendications 5 à 6 ou de la composition d'enrobage selon l'une quelconque des revendications 7 à 8 sur une plante, une semence végétale, un sillon d'ensemencement, un sol et/ou un milieu de croissance végétale, de préférence à une température inférieure à 15 °C, par exemple inférieure à 10 °C, de préférence entre 2 °C et 8 °C, plus préférablement autour de 5 °C.

**14.** Kit de parties pour stimuler la croissance végétale comprenant

- un premier récipient comprenant la bactérie ou la culture bactérienne biologiquement pure selon l'une quelconque des revendications 1 à 4, la composition selon l'une quelconque des revendications 5 à 6 et/ou la composition d'enrobage selon l'une quelconque des revendications 7 à 8 ; et
- des instructions pour appliquer la bactérie ou la culture bactérienne biologiquement pure selon l'une quelconque des revendications 1 à 4, la composition selon l'une quelconque des revendications 5 à 6 et/ou la composition d'enrobage selon l'une quelconque des revendications 7 à 8 sur des plantes, des semences végétales, ou un milieu de croissance végétale, de préférence à une température inférieure à 15 °C, par exemple inférieure à 10 °C, de préférence entre 2 °C et 8 °C, plus préférablement autour de 5 °C.

**15.** Bactérie ou culture bactérienne biologiquement pure selon l'une quelconque des revendications 1 à 4, composition selon l'une quelconque des revendications 5 à 6 ou composition d'enrobage selon l'une quelconque des revendications 7 à 8 pour une utilisation comme médicament.

Fig. 1

Fig. 2

Fig. 3

# Virulence factor analysis

Fig. 4

Fig. 5

Fig. 6A-6B

Fig. 6C

Fig. 7

**EP 4 650 436 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020214843 A1
- WO 2008130701 A1
- WO 2021180941 A1

**Non-patent literature cited in the description**

- **RAFIKOVA et al.** New Bacterial Strains of Pseudomonas laurentiana: Promising Agents for Agrobiotechnology. *MOSCOW UNIVERSITY BIOLOGICAL SCIENCES BULLETIN*, 2020, vol. 75 (4), 206-211
- **W.R PEARSON ; D.J. LIPMAN**. BLASTP program for protein alignment. 1988